(19) Europäisches Patentamt
European Patent Office
Office européen des brevets

(11) Veröffentlichungsnummer: 0 016 261
B1

(12) **EUROPÄISCHE PATENTSCHRIFT**

(45) Veröffentlichungstag der Patentschrift: **29.12.82**

(51) Int. Cl.³: **C 07 D 491/107**, A 61 K 31/445 // (C07D491/107, 221/00, 313/00)

(21) Anmeldenummer: **79105373.9**

(22) Anmeldetag: **27.12.79**

(54) **Neue Spiro(dibenz(b,f)oxepin-piperidine) und Verfahren zu ihrer Herstellung.**

(30) Priorität: **10.01.79 US 2348**

(43) Veröffentlichungstag der Anmeldung: **01.10.80 Patentblatt 80/20**

(45) Bekanntmachung des Hinweises auf die Patenterteilung: **29.12.82 Patentblatt 82/52**

(84) Benannte Vertragsstaaten: **AT BE CH DE FR GB IT NL SE**

(56) Entgegenhaltungen:
**AT-B-260 264**
**AT-B-306 036**
**DE-A-1 795 148**
**FR-M-8 456**
**US-A-4 001 418**

(73) Patentinhaber: **HOECHST AKTIENGESELLSCHAFT, Postfach 80 03 20, D-6230 Frankfurt/Main 80 (DE)**

(72) Erfinder: **Ong, Helen Hu, Bunker Hill Place 15, Whippany, New Jersey (US)**
Erfinder: **Profitt, James Arthur, Barclay Place 1805, Goshen, Indiana 46526 (US)**

EP 0 016 261 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Neue Spiro[dibenz(b,f)oxepin-piperidine] und Verfahren zu ihrer Herstellung

Vorliegende Erfindung betrifft neue Spiro[dibenz(b,f)oxepin-piperidine] sowie deren pharmazeutisch unbedenkliche Säureadditionssalze, welche als Analgetika, Tranquilizer und Anticonvulsiva nützlich sind und Arzneimittel, die eine solche Verbindung als wesentlichen Wirkstoff enthalten. Die erfindungsgemäßen Verbindungen entsprechen der Formel I

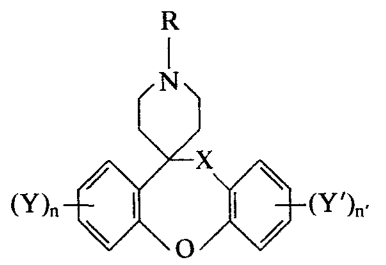

worin R für Wasserstoff, Alkyl, Alkenyl, Alkinyl, Hydroxyalkyl, Cycloalkylalkyl, gegebenenfalls substituiertes Phenylalkyl oder Phenoxyalkyl, Alkanoyl, gegebenenfalls substituiertes Benzoylalkyl, Cyan oder ein Äthylenglykolketal der Formel

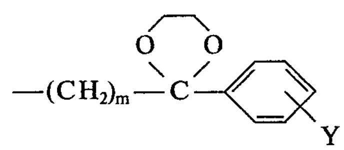

steht, X

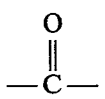 oder 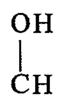

darstellt, wenn R die oben angegebene Bedeutung hat, oder

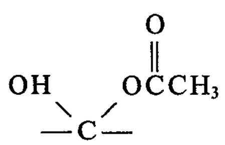

darstellt, wenn R für Alkanoyl, Methyl oder Cyan steht, Y und Y' gleich oder verschieden sind und je Wasserstoff, Chlor, Fluor, Brom, Methoxy, Methylthio oder Trifluormethyl bedeuten können, n und n' gleich oder verschieden sind und je eine ganze Zahl von 1 bis einschließlich 2 darstellen können und m eine ganze Zahl von 1 bis einschließlich 4 ist.

Bei den obigen Definitionen und überall in dieser Anmeldung haben die folgenden Begriffe die ihnen zugeschriebene Bedeutung:
»Alkyl« enthält 1 bis einschließlich 6 Kohlenstoffatome und kann geradkettig oder verzweigt sein;
»Alkenyl«, »Alkinyl« oder »Alkanoyl« können je 2 bis einschließlich 6 Kohlenstoffatome enthalten und geradkettig oder verzweigt sein;
»Cycloalkyl« enthält 3 bis einschließlich 7 Kohlenstoffatome, und
»substituiert« bedeutet, daß das Phenyl der jeweiligen Gruppe durch die folgenden Substituenten: Nitro, Amino, Chlor, Fluor, Brom, Methoxy, Niederalkyl (siehe oben stehende Definition) oder Trifluormethyl einfach oder mehrfach substituiert ist.

Die zur Herstellung der erfindungsgemäßen, pharmazeutisch unbedenklichen Säuresalze verwendbaren Säuren umfassen anorganische Säuren wie Salzsäure, Bromwasserstoffsäure, Schwefelsäure, Salpetersäure, Phosphorsäure und Überchlorsäure sowie organische Säuren wie Weinsäure, Zitronensäure, Essigsäure, Bernsteinsäure, Maleinsäure, Fumarsäure und Oxalsäure.

Die Verbindungen der vorliegenden Erfindung sind bisher nicht beschrieben worden. Galt u. a., in der US-Patentschrift 4 001 419, beschreiben als Analgetika 1'-substituierte Xanthen-9-spiro-4'-piperidinderivate der Formel

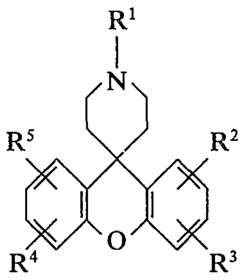

worin $R^1$ für ein Wasserstoffatom, einen Alkylrest mit 1 bis 10 Kohlenstoffatomen, einen Alkenylrest mit 3 bis 10 Kohlenstoffatomen, einen Halogenalkenylrest mit 3 bis 6 Kohlenstoffatomen, einen gegebenenfalls im Cycloalkylkern durch einen Arylrest mit 6 bis 10 Kohlenstoffatomen oder ein oder zwei Alkylreste mit 1 bis 3 Kohlenstoffatomen substituierten Cycloalkylalkylrest mit 4 bis 7 Kohlenstoffatomen, einen Phenylrest, einen gegebenenfalls im Arylkern durch eins bis drei Halogenatome oder Alkylreste mit 1 bis 3 Kohlenstoffatomen substituierten Arylalkylrest mit 7 bis 10 Kohlenstoffatomen, einen Hydroxyalkylrest mit 2 bis 5 Kohlenstoffatomen, einen Dialkylaminoalkylrest mit 4 bis 8 Kohlenstoffatomen, einen Carbamoylalkylrest mit 2 bis 8 Kohlenstoffatomen, einen Alkylcarbamoylalkylrest mit 3 bis 8 Kohlenstoffatomen oder einen Alkanoylalkylrest mit 3 bis 8 Kohlenstoffatomen steht, $R^2$, $R^3$, $R^4$ und $R^5$ gleich oder verschieden sind und je Wasserstoffatome, Halogenatome, Alkylreste mit 1 bis 5 Kohlenstoffatomen, Halogenalkylreste mit 1 bis 5 Kohlenstoffatomen, Alkoxyreste mit 1 bis 5 Kohlenstoffatomen, Alkylthioreste mit 1 bis 5 Kohlenstoffatomen, Hydroxylreste, Thiolreste, Alkanoylaminoreste mit 1 bis 5 Kohlenstoffatomen, Alkanoyloxyreste mit 1 bis 5 Kohlenstoffatomen, gegebenenfalls im Arylkern durch eins bis drei Halogenatome oder Alkylreste mit 1 bis 3 Kohlenstoffatomen substituierte Aroyloxyreste mit 7 bis 10 Kohlenstoffatomen, Arylalkenoyloxyreste mit 9 bis 12 Kohlenstoffatomen, Hydroxyalkylreste mit 1 bis 5 Kohlenstoffatomen, Alkylsulfinylreste mit 1 bis 5 Kohlenstoffatomen oder Alkansulfonylylreste mit 1 bis 5 Kohlenstoffatomen bedeuten können, sowie deren pharmazeutisch unbedenkliche Säureadditionssalze.

Ferner offenbaren Gerecke u. a., in der französischen Patentschrift 824 066, als Tranquilizer tricyclische Verbindungen der Formel

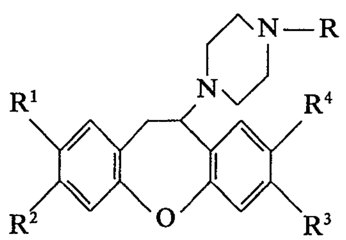

worin R eine Niederalkyl-, Niederalkenylalkyl- oder Niederalkinylalkylgruppe bedeutet, welche im Fall, wo sie von einer Methylgruppe verschieden ist, durch eine Cyan-, Hydroxyl- oder Alkanoyloxygruppe substituiert sein kann, oder worin R eine Gruppe der allgemeinen Formel

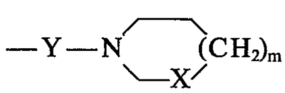

bedeutet,
in der X ein Sauerstoff- oder Schwefelatom, eine Iminogruppe, eine Niederalkyliminogruppe oder eine Methylengruppe und Y eine gegebenenfalls durch eine Niederalkylgruppe substituierte Äthylen- oder Propylengruppe darstellen, und m gleich 0 oder 1 ist, und ferner einer der Substituenten $R_1$ und $R_2$ für ein Wasserstoffatom und der andere für eine Niederalkyl-, Niederalkoxy- oder Niederalkylthiogruppe, ein Halogen oder eine Trifluormethyl- oder Hydroxylgruppe steht sowie einer der Substituenten $R_3$ und $R_4$ für Wasserstoff und der andere für ein Wasserstoffatom, eine Niederalkyl-, Niederalkoxy- oder Niederalkylthiogruppe, ein Halogen oder eine Trifluormethyl- oder Hydroxylgruppe steht, sowie Salze dieser Verbindungen.

Alkylpiperazinyl-10,11-dihydro-dibenzo[b,f]-thiepine und -oxepine sind aus den AT-Patentschriften 260 264 und 306 036 und aus der FR-Patentschrift 8 456 M bekannt. Die beschriebenen Verbindungen zeigen ebenfalls psychotrope und neurotrope Wirkungen, sind jedoch strukturell von den erfindungsgemäßen Verbindungen insofern deutlich verschieden, als sie keine Spiro-Verbindungen sind.

Ebenfalls nicht-spiro-Struktur haben die Alkyl-piperidyl- bzw. Alkyltetrahydropyridyl-10,11-dihydro-dibenzo[b,f]-thiepine bzw. -oxepine gemäß der DE-OS 1 795 148.

Ferner sind aus der US-Patentschrift 4 001 418 1-substituierte Thioxanthen-9-spiro-4′-piperidin-derivate mit analgetischer Wirkung bekannt. Diese Verbindungen besitzen zwar Spirostruktur, jedoch einen mittleren 6-Ring anstelle des 7-Rings der erfindungsgemäßen Verbindungen.

Die erfindungsgemäßen Verbindungen lassen sich nach einer oder mehreren der folgenden Methoden herstellen, wobei R, X, Y, Y' und Z die oben definierte Bedeutung haben, falls nicht anders angegeben.

Methode A

1. Man unterwirft ein 2-Phenoxyphenylacetonitril der Formel II

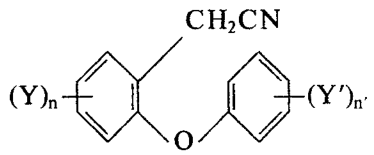

II

einer Bisalkylierung mit Mechloräthaminhydrochlorid in Gegenwart einer starken Base (als säurebindendes Mittel) bei einer Temperatur im Bereich von etwa Raumtemperatur bis etwa 85° C, wobei das entsprechende 4-Cyan-1-methyl-4-(2-phenoxyphenyl)-piperidin der Formel III

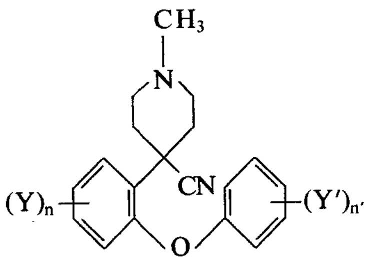

III

entsteht. Bevorzugte Arbeitsweisen schließen die Verwendung von Dimethylformamid oder Dimethylsulfoxyd als Lösungsmittel und von Natriumhydrid als säurebindendes Mittel ein.

2. Eine Verbindung der Formel III wird gemäß der ersten Stufe einer von Braunreaktion behandelt, was das entsprechende 1,4-Dicyan-4-(2-phenoxyphenyl)-piperidin der Formel IV

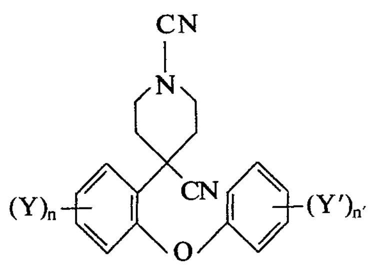

IV

liefert. Bei der Umsetzung wird Bromcyan zur Substitution des N-Methyls eingesetzt. Die Reaktionsbedingungen umfassen ferner ein Lösungsmittel wie Chloroform oder Methylenchlorid, eine als säurebindendes Mittel wirkende milde Base wie Kaliumcarbonat sowie eine Reaktionstemperatur im Bereich von etwa Raumtemperatur bis zum Rückfluß des Reaktionsgemischs.

3. Man unterwirft eine Verbindung der Formel IV einer sauren Hydrolyse, was die entsprechende 4-(2-Phenoxyphenyl)-piperidin-4-carbonsäure der Formel V

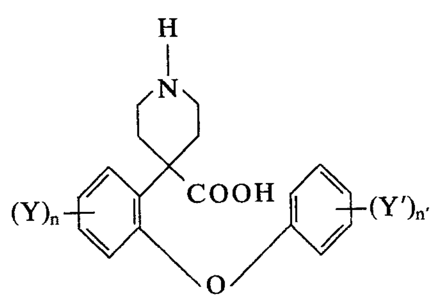

V

liefert. Bei einer bevorzugten Methode wird konzentrierte (48%) Bromwasserstoffsäure unter Rückflußbedingungen eingesetzt.

4. Eine Verbindung der Formel V wird in Gegenwart eines basischen Lösungsmittels oder eines säurebindenden Mittels acyliert, was die entsprechende 1-Alkanoyl-4-(2-phenoxyphenyl)-piperidin-4-carbonsäure der Formel VI

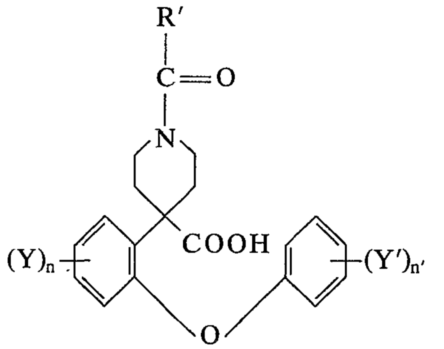

VI

worin R' für Alkyl steht, liefert. Vorzugsweise ist das Lösungsmittel Pyridin und das säurebindende Mittel Kaliumcarbonat. Das Acylierungsmittel kann ein geeignetes Alkanoylhalogenid, z. B. Acetylchlorid, oder ein Anhydrid, z. B. Essigsäureanhydrid, sein.

5. Eine Verbindung der Formel VI setzt man vorsichtig mit einem Thionylhalogenid um, was das entsprechende Säurehalogenid der Formel VII

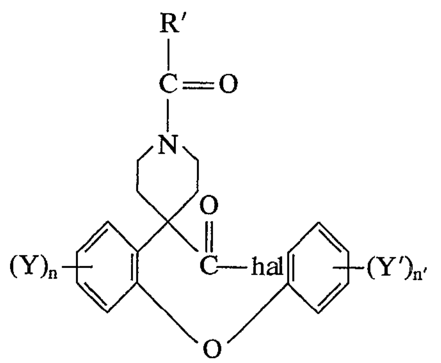

VII

liefert. Bei einer bevorzugten Arbeitsweise wird Thionylchlorid eingesetzt und die Reaktion durch etwa 5 Minuten Erhitzen auf dem Dampfbad durchgeführt.

6. Eine Verbindung der Formel VII wird cyclisiert, was das entsprechende 10,11-Dihydro-1'-alkanoyl-11-oxospiro[dibenz[b,f]oxepin-10,4'-piperidin], der Formel

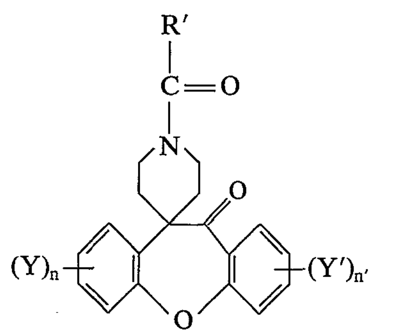

Ia

liefert. Diese Cyclisierung läßt sich über das Säurechlorid unter modifizierten Friedel-Craftsbedingungen mit einem Lewissäurekatalysator wie Aluminiumchlorid oder Eisen-III-chlorid durchführen. Methylenchlorid wird als Lösungsmittel bevorzugt. Die Reaktionstemperatur kann zwischen etwa 15°C und etwa 150°C liegen. Bei einer bevorzugten Arbeitsweise werden das Säurechlorid der Formel VII Aluminiumchlorid, Methylenchlorid und Rückflußbedingungen angewandt.

Methode B

1. Man unterwirft eine Verbindung der Formel III einer sauren Hydrolyse nach Methode A (3), was die entsprechende Carbonsäure der Formel

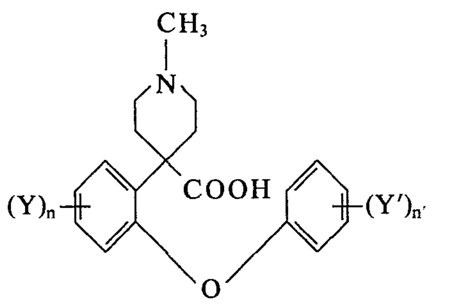

VIII

liefert.

2. Eine Verbindung der Formel VIII wird cyclisiert, was das entsprechende 10,11-Dihydro-1′-methyl-11-oxospiro[dibenz(b,f)oxepin-10,4′-piperidin], eine Verbindung der Formel

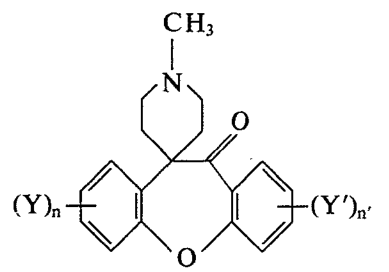

Ib

liefert. Bei einer bevorzugten Arbeitsweise verwendet man ein entwässerndes Medium wie Polyphosphorsäure und eine Reaktionstemperatur von etwa 95 – 115° C. Andernfalls läßt sich die Arbeitsweise der Methode A (6) anwenden.

Methode C

Man unterwirft eine Verbindung der Formel Ia einer sauren Hydrolyse, was die entsprechende erfindungsgemäße N-unsubstituierte Verbindung der Formel Ic

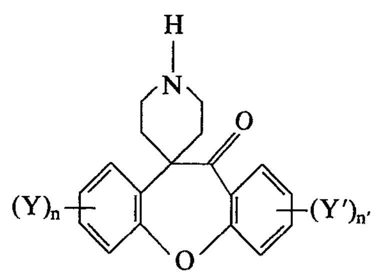

Ic

liefert. Die Behandlung mit 3n-Salzsäure unter Rückflußbedingungen stellt eine bevorzugte Arbeitsweise zur Durchführung dieser Hydrolyse dar.

Methode D

1. Man behandelt eine Verbindung der Formel Ib bzw. If weiter unten gemäß der Methode A (2), was die entsprechende erfindungsgemäße N-Cyanverbindung der Formel Id

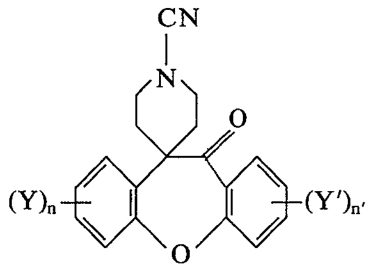

Id

liefert.

2. Eine Verbindung der Formel Id wird einer Hydrolyse gemäß der zweiten Stufe der von Braunreaktion unterworfen, was die entsprechende erfindungsgemäße N-unsubstituierte Verbindung der obigen Formel Ic liefert. Bei dieser Arbeitsweise werden ein Gemisch aus 3n-Salzsäure und Eisessig (2 : 1) sowie Temperaturen im Bereich von etwa 75 bis 150°C angewandt.

Methode E

Eine Verbindung der obigen Formeln Ia, Ib oder Id läßt sich unter Friedel-Craftsbedingungen acylieren, wie in Methode A (6) beschrieben, was die entsprechende erfindungsgemäße 11-Alkanoyloxy-11-hydroxyverbindung der Formel Ie

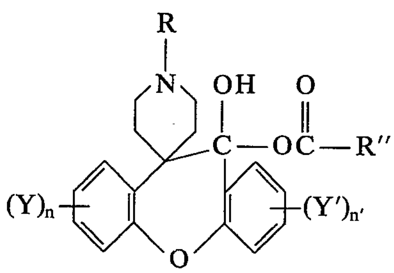

Ie

worin R

für 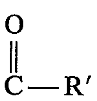

$CH_3$ oder CN steht sowie R′ und R″ gleich oder verschieden sind und je Alkyl bedeuten, liefert.

Methode F

Eine Verbindung der obigen Formeln Ia, Ib, Ic oder Id wird reduziert, was die entsprechende erfindungsgemäße 11-Hydroxyverbindung der Formel If

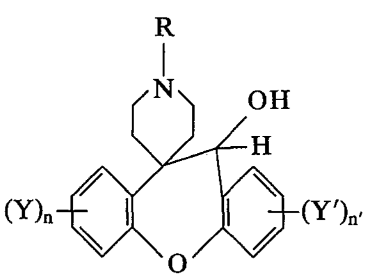

If

worin R für Alkyl oder H steht, liefert. Bei einer bevorzugten Arbeitsweise verwendet man

Lithiumaluminiumhydrid als Reduktionsmitte, Tetrahydrofuran als Lösungsmittel und eine Reaktionstemperatur im Bereich von Raumtemperatur bis zum Rückfluß des Reaktionsgemischs.

### Methode G

Eine Verbindung der Formel Ic oder If (R = H) wird nach einer geeigneten, dem Stand der Technik bekannten Methode mit einem entsprechenden Alkylierungsmittel alkyliert bzw. acyliert, was die entsprechende erfindungsgemäße N-substituierte Verbindung der Formel Ig

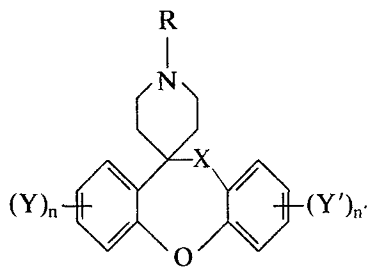

Ig

worin R für Alkyl, Hydroxyalkyl, Alkinyl, Alkenyl, Phenylalkyl, Phenoxyalkyl, Cycloalkylalkyl, Alkanoyl oder Benzoylalkyl und X für

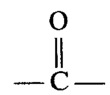 oder 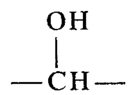

stehen, liefert. Bei bevorzugten Arbeitsweisen setzt man ein Alkylierungsmittel der Formel R – Halogenid ein, wobei die Alkylierung in einem Lösungsmittel wie Dimethylformamid, mit einem säurebindenden Mittel wie Natriumbicarbonat oder Kaliumcarbonat, einem Reaktionsinitiator wie Kaliumjodid und bei einer Reaktionstemperatur im Bereich von etwa Raumtemperatur bis zum Rückfluß des Reaktionsgemischs erfolgt.

### Methode H

1. Eine Verbindung der Formel Ic oder If (R = H) wird unter Bedingungen, die mit denen der Methode G übereinstimmen, mit einem Chloräthylenglykolketal der Formel

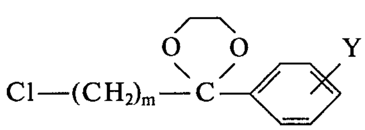

behandelt, was die entsprechende Verbindung der Formel I, worin R ein Benzoylalkyläthylenketal darstellt, liefert.
2. Man unterwirft eine erfindungsgemäße Äthylenketalverbindung einer sauren Hydrolyse, was die entsprechende erfindungsgemäße Verbindung, worin R Benzoylniederalkyl

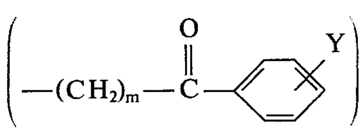

darstellt, liefert. Bei einer bevorzugten Arbeitsweise verwendet man 3n-Salzsäure in Äthanol als das die Hydrolyse bewirkende Mittel.

### Methode I

Man behandelt eine Verbindung der Formel Ic oder If (R = H) gemäß den Bedingungen der Eschweiler-Clarkereaktion, was die entsprechende Verbindung der Formel I, worin R Methyl darstellt, liefert. Diese Methode wird durch Umsetzung mit Ameisensäure und Formaldehyd unter Rückflußbedingungen durchgeführt.

Bei allen obigen Methoden sind die optimalen Bedingungen von den Ausgangsstoffen, Lösungsmitteln, Katalysatoren und sonstigen Reaktionsteilnehmern abhängig. Dies wird in den nachfolgenden Beispielen weiter erläutert.

Durch die Formel II dargestellte Ausgangsstoffe sind entweder im Handel erhältlich oder nach wohlbekannten Methoden gemäß dem folgenden Reaktionsschema herstellbar:

1.

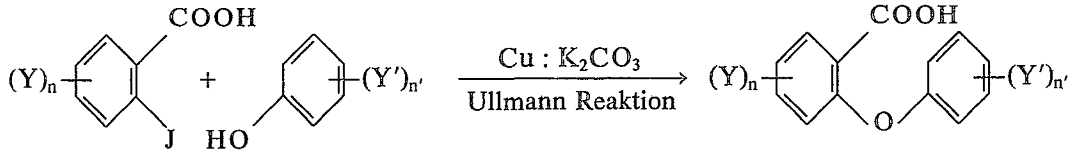


2.

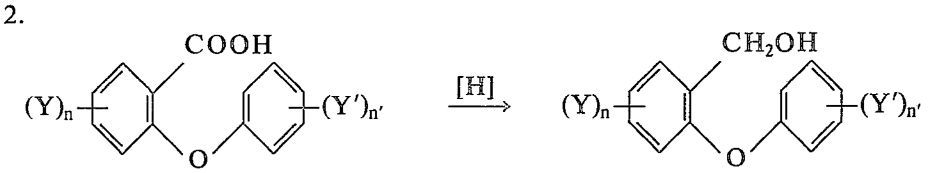


Reduktion mit Natrium-bis-(2-methoxyäthoxy)-aluminiumhydrid (VITRIDE®)

3.

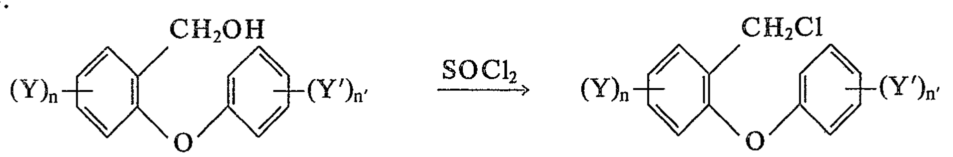


4.

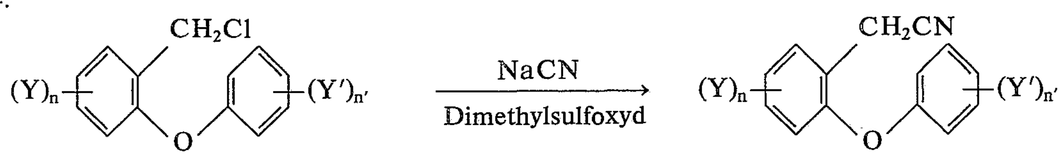


Die erfindungsgemäßen Verbindungen sind wegen ihrer Fähigkeit, bei Säugetieren Schmerzen zu lindern, als Analgetika wertvoll, wie beim durch Phenyl-α-chinon ausgelösten Krümmungssyndrom an der Maus, einem Standardtest auf Analgesie [Proc. Soc. Exptl. Biol. Med. 95, 729 (1957)], nachgewiesen. Die bei verschiedenen Dosierungen typischer erfindungsgemäßer Verbindungen erzielten prozentualen Hemmungen des Writhing sind unten in der Tabelle angegeben.

| Verbindung | Dosis mg/kg | % Hemmung |
|---|---|---|
| 2-Chlor-10,11-dihydro-1'-methyl-11-oxospiro [dibenz(b, f)oxepin-10,4'-piperidin] · HBr | 3,6 (s. c.)*)<br>5,2 (p. o.)**) | 50<br>50 |
| 2-Chlor-10,11-dihydro-11-oxospiro-[dibenz(b, f)oxepin-10,4'-piperidin] · HBr | 3,6 (s. c.)<br>7,7 (p. o.) | 50<br>50 |
| 10,11-Dihydro-11-oxo-1'-phenyl-äthyl-spiro[dibenz(b, f) oxepin-10,4'-piperidin] · HBr | 25 | 67 |
| 2-Chlor-10,11-dihydro-1'-äthyl-11-oxospiro[dibenz (b, f) oxepin-10,4'-piperidin] · HBr | 25 | 67 |

*) s. c. bedeutet subkutane Verabreichung.
**) p. o. bedeutet perorale Verabreichung.

Vergleichsweise bewirken Aspirin bzw. Propoxyphen, bekannte analgetische Mittel, eine 34%ige bzw. 50%ige Hemmung bei einer Dosis von 60 mg/kg bzw. 28 mg/kg. Diese Daten veranschaulichen, daß die erfindungsgemäßen Verbindungen bei Verabreichung in Tagesmengen von etwa 0,1 bis etwa 100 mg/kg Körpergewicht zur Linderung von Schmerzen in Säugetieren verwendbar sind.

Verbindungen der vorliegenden Erfindung sind ferner als Tranquilizer wertvoll, wie ihre Fähigkeit, Aggressivität nach Pfotenschock zu hemmen, zeigt [Arch. Int. Pharmacodynam. et de Therap. 142, 30 (1963)]. Beispielsweise bieten 10,11-Dihydro-1′-[3-(4-fluorbenzoyl)-propyl]-11-oxospiro[dibenz(b,f)oxepin-10,4′-piperidin] · HBr bzw. 10,11-Dihydro-11-oxo-1′-(2-propinyl)-spiro[dibenz(b,f)oxepin-10,4′-piperidin] · HBr in einer peroralen Dosis von 20 mg/kg an der Maus einen 100%igen bzw. 50%igen Schutz gegen Aggressivität nach Pfotenschock. Diese Verwendbarkeit der erfindungsgemäßen Verbindungen als Tranquilizer folgt auch aus ihrer Fähigkeit bei Aggregationszuständen der Toxizität von Amphetamin an der Maus entgegenzuwirken [J. Pharmacol. Exp. Therap. 87, 214 (1946)]. Beispielsweise wirkt eine 50 mg/kg-Dosis von 2-Chlor-10,11-dihydro-11-oxospiro[dibenz(b,f)oxepin-10,4′-piperidin] · HBr der Amphetamintoxizität bei 50% der Mäuse entgegen. Diese Daten veranschaulichen, daß die erfindungsgemäßen Verbindungen bei Verabreichung in Tagesmengen im Bereich von etwa 0,5 bis etwa 100 mg/kg Körpergewicht in Säugetieren als Tranquilizer verwendbar sind.

Verbindungen der vorliegenden Erfindung sind ferner als Krampfmittel für Säugetiere wertvoll, wie nach L. A. Woodbury und V. D. Davenport [Arch. Int. Pharmacodynam. 92, S. 97 – 107 (1952)] bestimmt. Beispielsweise bietet eine intraperitoneale Dosis von 19,4 mg/kg Körpergewicht von 2-Chlor-10,11-dihydro-1′-methyl-11-oxospiro[dibenz(b,f)oxepin-10,4′-piperidin] · HBr einen 50%igen Schutz gegen die Wirkung eines über dem Maximum liegenden Elektroschocks. Ähnliche Wirkungen erreicht man mit intraperitonealen Dosierungen von 27,1 bzw. 30 – 50 mg/kg von 2-Chlor-1′-cyclopropylmethyl-10,11-dihydro-11-oxospiro[dibenz(b,f)oxepin-10,4′-piperidin] · HBr bzw. 10,11-Dihydro-1′-methyl-11-oxospiro[dibenz(b,f)oxepin-10,4′-piperidin] · HBr. Diese Daten veranschaulichen, daß die erfindungsgemäßen Verbindungen bei Verabreichung in Tagesmengen im Bereich von etwa 0,5 bis 100 mg/kg Körpergewicht zur Behandlung von Krämpfen bei Säugetieren verwendbar sind.

Erfindungsgemäße Verbindungen sind unter anderem:

8-Chlor-10,11-dihydro-2-methoxy-1′-methyl-11-oxospiro-[dibenz(b,f)oxepin-10,4′-piperidin],
3-Brom-10,11-dihydro-7-methylthio-1′-methyl-11-oxospiro-[dibenz(b,f)oxepin-10,4′-piperidin],
10,11-Dihydro-3-methoxy-1′-methyl-11-oxo-8-trifluormethylspiro-[dibenz(b,f)oxepin-10,4′-piperidin],
10,11-Dihydro-9-methoxy-1′-methyl-2-methyl-11-oxospiro-[dibenz(b,f)oxepin-10,4′-piperidin],
10,11-Dihydro-1′-methyl-8-methyl-11-oxo-2-trifluormethylspiro-[dibenz(b,f)oxepin-10,4′-piperidin],
2-Brom-7-chlor-10,11-dihydro-1′-methyl-11-oxospiro-[dibenz(b,f)oxepin-10,4′-piperidin],
10,11-Dihydro-2,8-dimethoxy-1′-methyl-11-oxospiro-[dibenz(b,f)oxepin-10,4′-piperidin],
10,11-Dihydro-1′-methyl-2-methylthio-11-oxo-7-trifluormethylspiro-[dibenz(b,f)oxepin-10,4′-piperidin],
2-Fluor-1′-[2-(p-fluorbenzoyl)-äthyl]-10,11-dihydro-11-oxospiro-[dibenz(b,f)oxepin-10,4′-piperidin],
2-Chlor-1′-[2-(p-fluorbenzoyl)-äthyl]-10,11-dihydro-11-oxospiro-[dibenz(b,f)oxepin-10,4′-piperidin],
1′-(4-Benzoylbutyl)-10,11-dihydro-11-oxospiro-[dibenz(b,f)oxepin-10,4′-piperidin],
1′-(4-Benzoylbutyl)-2-chlor-10,11-dihydro-11-oxospiro-[dibenz(b,f)oxepin-10,4′-piperidin],
1′-(4-Benzoylbutyl)-2-fluor-10,11-dihydro-11-oxospiro-[dibenz(b,f)oxepin-10,4′-piperidin],
2-Fluor-10,11-dihydro-7,8-dimethoxy-1′-methyl-11-oxospiro-[dibenz(b,f)oxepin-10,4′-piperidin],
2,3-Dichlor-10,11-dihydro-1′-methyl-7-methylthio-11-oxospiro-[dibenz(b,f)oxepin-10,4′-piperidin],
8-Chlor-2,3-difluor-10,11-dihydro-1′-methyl-11-oxospiro-[dibenz(b,f)oxepin-10,4′-piperidin] und
2-Chlor-7,8-difluor-10,11-dihydro-1′-methyl-11-oxospiro-[dibenz(b,f)oxepin-10,4′-piperidin].

Wirksame Mengen der erfindungsgemäßen Verbindungen können einem Patienten nach

irgendeiner von verschiedenen Methoden verabreicht werden, beispielsweise peroral wie in Kapseln oder Tabletten, parenteral in Form steriler Lösungen oder Suspensionen sowie in einigen Fällen intravenös in Form steriler Lösungen. Die Endprodukte selbst sind zwar als freie Base wirksam, doch kann man sie aus Gründen der Stabilität, Leichtigkeit der Kristallisation, erhöhter Löslichkeit und dergleichen in Form ihrer pharmazeutisch unbedenklichen Additionssalze formulieren und verabreichen.

Die erfindungsgemäßen Wirkstoffe lassen sich peroral verabreichen, beispielsweise mit einem inerten Streckmittel oder eßbaren Träger, oder sie können in Gelatinekapseln eingeschlossen oder zu Tabletten komprimiert werden. Zur peroralen therapeutischen Verabreichung kann man die erfindungsgemäßen Wirkstoffe in Vehikel einarbeiten und in Form von Tabletten, Pastillen, Kapseln, Elixieren, Suspensionen, Sirupen, Oblaten, Kaugummi und dergleichen anwenden. Diese Präparate sollen mindestens 0,5% Wirkstoff enthalten, aber dies läßt sich in Abhängigkeit von der jeweiligen Form variieren und kann zweckmäßig zwischen 4% und etwa 70% des Einheitsgewichts liegen. Eine solche Wirkstoffmenge liegt in derartigen Zusammensetzungen vor, daß sich eine geeignete Dosis ergibt. Bevorzugte Zusammensetzungen und Präparate gemäß vorliegender Erfindung werden so zubereitet, daß eine perorale Dosiereinheitsform 1,0–300 Milligramm Wirkstoff enthält.

Die Tabletten, Pillen, Kapseln, Pastillen und dergleichen können ferner folgende Bestandteile enthalten: ein Bindemittel wie mikrokristalline Cellulose, Traganthgummi oder Gelative; ein Vehikel wie Stärke oder Milchzucker, ein Sprengmittel wie Alginsäure, Primogel, Maisstärke und dergleichen; ein Schmiermittel wie Magnesiumstearat oder Sterotex, ein Gleitmittel wie kolloidales Siliciumdioxyd; ferner kann man einen Süßstoff wie Rohrzucker oder Saccharin oder auch einen Geschmacksstoff wie Pfefferminz, Methylsalicylat oder Orangenaroma zusetzen. Ist die Dosiereinheitsform eine Kapsel, so kann diese neben Stoffen der obigen Art einen flüssigen Träger wie ein fettes Öl enthalten. Andere Dosiereinheitsformen können weitere verschiedene Stoffe enthalten, welche die physikalische Form der Dosiereinheit modifizieren, wie beispielsweise Überzüge. Tabletten oder Pillen können somit mit Zucker oder Schellack überzogen oder mit anderen, erst im Darm löslichen Überzügen versehen werden. Ein Sirup kann neben den Wirkstoffen Rohrzucker als Süßstoff sowie gewisse Konservierungsmittel, Farbstoffe und Farben sowie Geschmacksstoffe enthalten. Bei der Bereitung dieser verschiedenen Zusammensetzungen verwendete Stoffe sollen pharmazeutisch rein und in den verwendeten Mengen nicht toxisch sein.

Zur parenteralen therapeutischen Verabreichung können die erfindungsgemäßen Wirkstoffe in eine Lösung oder Suspension eingearbeitet werden. Diese Zubereitungen sollen mindestens 0,1% Wirkstoff enthalten, doch läßt sich dies zwischen 0,5 und etwa 50% ihres Gewichtes variieren. Eine solche Wirkstoffmenge liegt in derartigen Zusammensetzungen vor, daß sich eine geeignete Dosis ergibt. Bevorzugte Zusammensetzungen und Präparate gemäß vorliegender Erfindung werden so hergestellt, daß eine parenterale Dosiereinheit 0,5 bis 100 Milligramm Wirkstoff enthält.

Die Lösungen oder Suspensionen können ferner folgende Bestandteile umfassen: ein steriles Verdünnungsmittel wie Wasser zur Injektion, physiologische Kochsalzlösung, nichtflüchtige Öle, Polyäthylenglykole, Glycerin, Propylenglykol oder sonstige synthetische Lösungsmittel; antibakterielle Mittel wie Benzylalkohol oder Methylparaben; Antioxydantien wie Ascorbinsäure oder Natriumbisulfit; Chelatbildner wie Äthylendiamintetraessigsäure; Puffer wie Acetate, Citrate oder Phosphate sowie Mittel zur Einstellung des osmotischen Drucks, wie Kochsalz oder Dextrose. Das parenterale Präparat kann in Ampullen, zum einmaligen Gebrauch bestimmten Spritzen oder Mehrfachdosis-Phiolen aus Glas oder Kunststoff eingeschmolzen werden.

## Beispiel 1

a) Man gibt 2,6 g 99%iges Natriumhydrid portionsweise zu einer Lösung von 4,4 g 2-Phenoxyphenylacetonitril in 50 ml Dimethylformamid. Das dabei gebildete rötlichbraune Gemisch wird 30 Minuten gerührt, bevor man eine Lösung von 4,0 g Mechloräthaminhydrochlorid in 50 ml Dimethylformamid dazutropft, wobei man die Gasentwicklungsgeschwindigkeit niedrig hält. Nach beendeter Zugabe wird das Gemisch 15 Stunden bei 80–85°C gerührt. Danach gibt man 100 g Eis dazu, bevor das Gemisch dreimal mit Äther extrahiert wird. Die vereinigten Ätherextrakte trocknet man über Kaliumcarbonat und entfernt dann das Lösungsmittel im Vakuum, wobei ein bräunliches Öl verbleibt. Das Öl löst man in einer kleinen Menge Äther und läßt es mit weiterem Äther durch eine in Äther gefüllte Aluminiumoxydsäule laufen. Die filtrierte Lösung wird eingeengt, wobei ein schweres Öl zurückbleibt. Das Öl wird in sein Hydrochlorid umgewandelt und dieses aus Aceton/Äther umkristallisiert, wobei man 4-Cyan-1-methyl-4-(2-phenoxyphenyl)-piperidinhydrochlorid als glänzende Prismen vom Schmelzpunkt 242–244°C erhält.

b) Unter Rühren gibt man eine Lösung von 2,6 g 4-Cyan-1-methyl-4-(2-phenoxyphenyl)-piperidin, der freien Base aus a, in 22 ml Chloroform rasch zu einem Gemisch aus 1,5 g Bromcyan und 6 g Kaliumcarbonat in 30 ml Chloroform. Es wird 16 Stunden unter Rückfluß gerührt und danach filtriert. Das Filtrat wird zur Trockne eingeengt, was einen öligen Rückstand hinterläßt, den man in einigen ml Methylalkohol auflöst, um gegebenenfalls nicht umgesetztes Bromcyan zu zersetzen. Der

Methylalkohol wird dann abgedampft, wobei ein beim Abkühlen kristallisierender blaßgelblicher Rückstand verbleibt. Der entstandene Feststoff wird aus Aceton umkristallisiert, wobei man 1,4-Dicyan-4-(2-phenoxyphenyl)-piperidin als rhombische Kristalle vom Schmelzpunkt 100–101°C erhält.

c) Man rührt eine Suspension von 8 g 1,4-Dicyan-4-(2-phenoxyphenyl)-piperidin in 120 ml 48%iger Bromwasserstoffsäure 16 Stunden unter Rückfluß. Überschüssige Säure wird dann durch Vakuumdestillation entfernt, wobei ein glasiger Rückstand verbleibt, den man in 100 ml Wasser auflöst. Die Lösung trübt sich, Kristalle beginnen sich abzuscheiden und das trübe Gemisch wird 16 Stunden lang bei 0°C gehalten, wobei vollständige Kristallisation eintritt. Die Kristalle werden gesammelt und dann aus Äthylalkohol/Aceton/Äther umkristallisiert, wobei man 4-(2-Phenoxyphenyl)-piperidin-4-carbonsäurehydrobromid als hellbräunliche Prismen vom Schmelzpunkt 247–249°C erhält.

d) Man erhitzt ein Gemisch aus 8 g 4-(2-Phenoxyphenyl)-piperidin-4-carbonsäurehydrobromid und 10 ml Eissigsäureanhydrid in 50 ml Pyridin 4 Stunden zum Rückfluß. Überschüssiges Pyridin wird dann durch Vakuumdestillation entfernt und der Rückstand mit 100 ml 1n-Salzsäure angerieben und dann dreimal mit Chloroform extrahiert. Die vereinigten Chloroformextrakte werden nacheinander mit Wasser gut gewaschen, getrocknet und im Vakuum eingeengt, wobei ein fester Rückstand verbleibt. Dieser wird aus siedendem Aceton umkristallisiert, wobei man 1-Acetyl-4-(2-phenoxyphenyl)-piperidin-4-carbonsäure als kleine Prismen vom Schmelzpunkt 195–197,5°C erhält.

e) Man erhitzt eine Suspension von 3,4 g 1-Acetyl-4-(2-phenoxyphenyl)-piperidin-4-carbonsäure in 5 ml frisch destilliertem Thionylchlorid 5 Minuten auf dem Dampfbad, wobei sich eine klare Lösung ergibt. Überschüssiges Thionylchlorid wird unter vermindertem Druck bei 50°C entfernt, wobei eine gelbliche feste Masse verbleibt. Diese wird fünfmal aus Benzol/Cyclohexan umkristallisiert, wobei man 1-Acetyl-4-(2-phenoxyphenyl)-piperidin-4-carbonylchlorid als Produkt vom Schmelzpunkt 106–108°C erhält.

f) Man gibt eine Lösung von 14 g 1-Acetyl-4-(2-phenoxyphenyl)-piperidin-4-carbonylchlorid in 500 ml Methylenchlorid langsam unter Rühren zu 7,5 g Aluminiumchlorid. Nach beendeter Zugabe erhitzt man 2 Stunden zum Rückfluß, was zu einer Farbänderung von grünlich auf hellbräunlich führt. Anschließend wird Eis zugesetzt, bevor man dreimal mit Chloroform extrahiert. Die vereinigten Chloroformextrakte werden nacheinander mit verdünntem Alkali und mit Wasser gewaschen, getrocknet und im Vakuum vom Lösungsmittel befreit, wobei ein beim Stehen erstarrender öliger Rückstand verbleibt. Der Feststoff wird aus Benzol/Hexan umkristallisiert, wobei man 10,11-Dihydro-1'-acetyl-11-oxospiro[dibenz(b,f)oxepin-10,4'-piperidin] als farblose Prismen vom Schmelzpunkt 171–172°C erhält.

Analyse:

| | | | |
|---|---|---|---|
| Berechnet für $C_{20}H_{19}NO_3$: | 74,73% C; | 5,96% H; | 4,35% N. |
| Gefunden: | 74,93% C; | 6,02% H; | 4,43% N. |

## Beispiel 2

a) Man gibt 14,4 g 97%iges Natriumhydrid in kleinen Portionen unter Rühren zu einem Gemisch aus 27,6 g 2-(4-Chlorphenoxy)-benzylcyanid und 300 ml Dimethylformamid. Nach beendeter Zugabe wird 45 Minuten bei Raumtemperatur gerührt, bevor man 20,2 g 98%iges Mechloräthaminhydrochlorid in 200 ml Dimethylformamid dazutropft. Anschließend rührt man das Reaktionsgemisch 17 Stunden bei 100°C und läßt abkühlen. Das abgekühlte Gemisch wird auf 1000 g Eis gegossen und dann umgerührt. Das wäßrige Gemisch extrahiert man fünfmal mit je 250 ml Äther. Die vereinigten Ätherextrakte werden mit 2n-Salzsäure extrahiert und die vereinigten sauren Extrakte mit konzentrierter Ammoniumhydroxydlösung basisch gemacht, was ein Öl liefert. Dieses extrahiert man dreimal mit je 250 ml Äther und bringt die vereinigten Ätherextrakte zur Trockne, was eine dunkle kristalline Substanz ergibt. Der Feststoff wird auf einer Aluminiumoxydsäule mit Äther als Eluiermittel chromatographiert, wobei man 1-Methyl-4-cyan-4-[2-(4-chlorphenoxy)-phenyl]-piperidin als weißes kristallines Produkt vom Schmelzpunkt 95–97°C erhält.

b) Man rührt eine Lösung von 1,1 g 1-Methyl-4-cyan-4-[2-(4-chlorphenoxy)-phenyl]-piperidin in 25 ml 48%iger Bromwasserstoffsäure 16 Stunden bei 130°C. Danach verdünnt man mit 175 ml Wasser und dampft am Rotationsverdampfer unter vermindertem Druck bei 85°C ein, um überschüssige Säure zu entfernen. Der entstandene glasige Feststoff wird in Wasser aufgelöst und mit 1,5n-Ammoniumhydroxyd als Eluiermittel durch eine Bio-Rad AG 50 W-X8-Kationenaustauscherharzsäule laufengelassen, wobei man 4-[2-(4-Chlorphenoxy)-phenyl]-1-methylpiperidin-4-carbonsäure als weißes Pulver vom Schmelzpunkt 320°C erhält.

Analyse:

| | | | | |
|---|---|---|---|---|
| Berechnet für $C_{19}H_{20}ClNO_3$: | 65,99% C; | 5,83% H; | 4,05% N; | 10,25% Cl. |
| Gefunden: | 65,80% C; | 5,68% H; | 3,93% N; | 10,50% Cl. |

c) Man rührt ein Gemisch aus 4,4 g 4-[2-(4-Chlorphenoxy)-phenyl]-1-methylpiperidin-4-carbonsäure und 40 ml Polyphosphorsäure unter Stickstoff 2 Stunden bei 100 – 110° C. Danach kühlt man auf 0° C ab und versetzt mit 10 ml Eis und 70 ml Wasser. Das Gemisch wird mit konzentrierter Ammoniumhydroxydlösung alkalisch gemacht und viermal mit je 100 ml Äther extrahiert. Die vereinigten Ätherextrakte wäscht man nacheinander mit 100 ml Wasser, 100 ml 10%iger Natronlauge und 50 ml gesättigter Kochsalzlösung. Nach dem Waschen wird die Lösung zur Trockne gebracht, was ein Öl liefert. Dieses wird auf einer Silikagel-60-säule mit Methylalkohol/Chloroform (1 : 6) als Eluiermittel chromatographiert, was ein reineres Öl ergibt, welches man in Äther in sein Hydrobromidsalz überführt. Dieses wird aus Methylalkohol/Äther umkristallisiert, wobei man 2-Chlor-10,11-dihydro-1'-methyl-11-oxospiro[dibenz(b,f)oxepin-10,4'-piperidin]-hydrobromid als weißen Feststoff vom Schmelzpunkt 304 – 306° C erhält.

Analyse:
Berechnet für $C_{19}H_{18}ClNO_2 \cdot HBr$: 55,87% C; 4,69% H; 3,43% N; 19,57% Br.
Gefunden: 55,45% C; 4,97% H; 3,25% N; 19,66% Br.

Beispiel 3

Man rührt eine Suspension von 2,0 g 10,11-Dihydro-1'-acetyl-11-oxospiro[dibenz(b,f)oxepin-10,4'-piperidin], Beispiel 1, in 15 ml 3n-Salzsäure 3 Stunden unter Rückfluß. Die entstandene klare Lösung wird mit kaltem Wasser verdünnt und dann mit Essigester extrahiert. Die saure wäßrige Lösung macht man mit Kaliumcarbonat alkalisch, wobei ein Amin freigesetzt wird, das man in Äther auflöst. Die Ätherlösung wird nacheinander getrocknet und im Vakuum eingeengt, wobei ein blaßgelbliches Öl verbleibt, welches man in Äther in sein Bromwasserstoffsäuresalz überführt. Dieses wird aus Methylalkohol/Aceton/Äther umkristallisiert, wobei man 10,11-Dihydro-11-oxospiro[dibenz(b,f)oxepin-10,4'-piperidin]-hydrobromid als Nadeln vom Schmelzpunkt 280 – 282° C erhält.

Analyse:
Berechnet für $C_{18}H_{17}NO_2 \cdot HBr$: 60,02% C; 5,03% H; 3,88% N; 22,18% Br.
Gefunden: 60,02% C; 5,10% H; 3,77% N; 22,31% Br.

Beispiel 4

Man erhitzt ein Gemisch aus 3,5 g 2-Chlor-10,11-dihydro-1'-methyl-11-oxospiro[dibenz(b,f)oxepin-10,4'-piperidin], der freien Base aus Beispiel 2, 7,1 g Kaliumcarbonat und 1,8 g Bromcyan in 45 ml Methylenchlorid 2 Stunden unter Rückfluß. Dann filtriert man und dampft das Filtrat am Rotationsverdampfer ein, wobei ein Feststoff verbleibt. Dieser wird in 30 ml Methylalkohol aufgelöst und die Lösung 10 Minuten am Rückfluß erhitzt und dann wiederum am Rotationsverdampfer eingedampft, wobei ein Feststoff verbleibt. Diesen chromatographiert man mit Äther über 50 g Silikagel 60, wobei ein nicht-kristalliner Feststoff verbleibt, den man in Chloroform löst. Die Chloroformlösung wird am Rotationsverdampfer eingedampft, wobei ein bröckliger Schaum verbleibt, den man leicht zu einem weißen Pulver aufbrechen kann. Das Pulver wird aus Aceton umkristallisiert, wobei man 2-Chlor-1'-cyan-10,11-dihydro-11-oxospiro[dibenz(b,f)oxepin-10,4'-piperidin] als Produkt vom Schmelzpunkt 141,5 – 143,0° C erhält.

Analyse:
Berechnet für $C_{19}H_{15}ClN_2O_2$: 67,36% C; 4,46% H; 8,27% N.
Gefunden: 67,43% C; 4,65% H; 8,19% N.

Beispiel 5

Man rührt eine homogene Lösung von 1,8 g 2-Chlor-1'-cyan-10,11-dihydro-11-oxospiro[dibenz(b,f)oxepin-10,4'-piperidin], Beispiel 4, in 20 ml 3n-Salzsäure und 10 ml Eisessig 16 Stunden bei 110 – 120° C und läßt dann auf Raumtemperatur abkühlen. Die Lösung wird anschließend mit 50 ml Wasser verdünnt und dann bei 80° C am Rotationsverdampfer bis zur Entfernung von 50 ml Wasser eingedampft, worauf man den Verdünnungs- und Verdampfungsschritt wiederholt. Der Rückstand wird mit 200 ml Wasser verdünnt und die verdünnte Lösung nacheinander im Eisbad gekühlt, mit einer kleinen Menge 58%igem Ammoniumhydroxyd auf pH 9 basisch gemacht und dreimal mit je 80 ml Äther extrahiert. Die vereinigten Ätherextrakte wäscht man nacheinander zweimal mit je 80 ml Wasser und einmal mit 40 ml gesättigtem Kochsalz, trocknet dann über Magnesiumsulfat und dampft schließlich zur Trockne ein, wobei ein Öl verbleibt. Dieses löst man in Äther und überführt es in sein Bromwasserstoffsäuresalz. Dieses wird aus Aceton umkristallisiert, wobei man 2-Chlor-10,11-dihydro-11-oxospiro[dibenz(b,f)oxepin-10,4'-piperidin]-hydrobromid als Kristalle erhält, welche beim Erhitzen über 125° C ihre reguläre Gestalt verlieren, wobei das entstandene Glas bei 223–225° C schmilzt.

Analyse:
Berechnet für $C_{18}H_{16}ClNO_2 \cdot HBr$: 54,77% C; 4,34% H; 3,55% N; Gesamt-Hal 29,2%.
Gefunden: 54,69% C; 4,47% H; 3,39% N; Gesamt-Hal 29,0%.

Beispiel 6

Im Verlauf von 1–2 Minuten gibt man eine Lösung von 0,[illegible] g Acetylchlorid in Methylenchlorid unter Rühren zu einem Gemisch aus 1,0 g 10,11-Dihydro-1'-acetyl-11-oxospiro[dibenz(b,f)oxepin-10,4'-piperidin], Beispiel 1, und 0,94 g Aluminiumchlorid in 20 ml Methylenchlorid. Nach beendeter Zugabe rührt man 16 Stunden bei Raumtemperatur und zersetzt d[illegible] mit Eiswasser. Anschließend wird die organische Phase dreimal mit Essigester/Äther (1 : 1) extrahiert, nacheinander mit Natriumbicarbonatlösung, Wasser und Kochsalzlösung gewaschen, getrocknet und im Vakuum eingeengt, wobei ein gelbliches Öl verbleibt, das beim Kratzen kristallisiert. Der entstandene Feststoff wird aus Aceton/Hexan umkristallsiert, wobei man 10,11-Dihydro-11-acetoxy-1'-acetyl-11-hydroxyspiro[dibenz(b,f)oxepin-10,4'-piperidin] als Prismen vom Schmelzpunkt 193–196° C erhält.

Analyse:
Berechnet für $C_{22}H_{23}NO_5$: 69,29% C; 6,08% H; 3,65% N.
Gefunden: 69,28% C; 6,15% H; 3,58% N.

Beispiel 7

Man tropft eine Lösung von 2,4 g 10,11-Dihydro-11-oxospiro[dibenz(b,f)oxepin-10,4'-piperidin], der freien Base aus Beispiel 3 in 20 ml Tetrahydrofuran zu einer am Rückfluß erhitzten Aufschlämmung von 1,5 g Lithiumaluminiumhydrid in 20 ml Tetrahydrofuran. Nach beendeter Zugabe rührt man noch weitere 4 Stunden unter Rückfluß und dann 16 Stunden bei Raumtemperatur. Anschließend wird das Reaktionsgemisch durch aufeinanderfolgende Zugabe von 1,7 ml Wasser mit 1,7 ml 15%iger Natronlauge und 5,2 ml Wasser zersetzt. Das verdünnte Gemisch wird filtriert und das Filtrat im Vakuum zu einem festen Rückstand eingeengt. Der Rückstand wird aus siedendem Aceton umkristallisiert, wobei man 10,11-Dihydro-11-hydroxyspiro[dibenz(b,f)oxepin-10,4'-piperidin] als farblose Prismen vom Schmelzpunkt 202–203,5° C erhält.

Analyse:
Berechnet für $C_{18}H_{19}NO_2$: 76,86% C; 6,80% H; 4,97% N.
Gefunden: 76,77% C; 6,83% H; 4,96% N.

Beispiel 8

Man erhitzt ein Gemisch aus 1,1 g 10,11-Dihydro-11-oxospiro[dibenz(b,f)oxepin-10,4'-piperidin], der freien Base aus Beispiel 3 in 8 ml Ameisensäure 2 Stunden zum Rückfluß und setzt dann 7,2 ml Formalin (38% Formaldehyd in Wasser) dazu. Nach der Zugabe wird 16 Stunden weitergerührt und am Rückfluß erhitzt. Anschließend wird das Reagenzgemisch bei vermindertem Druck erwärmt und der Rückstand mit verdünntem Alkali basisch gemacht. Ein Öl scheidet sich ab und wird mit Äther extrahiert. Man trocknet die vereinigten Ätherextrakte und dampft dann im Vakuum ein, was ein farbloses Öl ergibt. Dieses wird in Äther gelöst und in sein Bromwasserstoffsäuresalz überführt, welches aus Methylalkohol/Äther umkristallisiert wird, wobei man 10,11-Dihydro-1'-methyl-11-oxospiro[dibenz(b,f)oxepin-10,4'-piperidin]-hydrobromid als Mikrogranulat vom Schmelzpunkt $>$290° C erhält.

Analyse:
Berechnet für $C_{19}H_{19}NO_2 \cdot HBr$: 60,96% C; 5,38% H; 3,74% N; 21,35% Br.
Gefunden: 61,14% C; 5,55% H; 3,84% N; 21,17% Br.

Beispiel 9

Man behandelt eine Lösung von 5,0 g 10,11-Dihydro-1'-methyl-11-oxospiro[dibenz(b,f)oxepin-10,4'-piperidin], der freien Base aus Beispiel 8 in 40 ml Tetrahydrofuran gemäß der Arbeitsweise von Beispiel 7, was einen Feststoff ergibt. Dieser wird aus Aceton/Hexan umkristallisiert, wobei man 10,11-Dihydro-11-hydroxy-1'-methylspiro[dibenz(b,f)oxepin-10,4'-piperidin] als farblose Prismen vom

Schmelzpunkt 180 – 182° C (Zersetzung) erhält.

Analyse:
Berechnet für $C_{19}H_{21}NO_2$: 77,25% C; 7,16% H; 4,74% N.
Gefunden: 77,22% C; 7,23% H; 4,67% N.

Beispiel 10

Man rührt ein Gemisch aus 1,0 g 10,11-Dihydro-11-oxospiro[dibenz(b,f)oxepin-10,4'-piperidin], der freien Base aus Beispiel 3, 0,3 g 2-Propinylchlorid und 1 g Natriumbicarbonat in 15 ml Dimethylformamid 16 Stunden bei 70° C. Danach wird das Gemisch nacheinander mit Äther verdünnt, filtriert, mit Wasser gewaschen, getrocknet und im Vakuum eingeengt, wobei ein blaßgelbliches Öl verbleibt. Dieses überführt man in Äther in sein Bromwasserstoffsäuresalz, welches aus Methylalkohol/Aceton/Äther umkristallisiert wird, wobei man 10,11-Dihydro-11-oxo-1'-(2-propinyl)-spiro[dibenz(b,f)oxepin-10,4'-piperidin]-hydrobromid als farbloses Granulat vom Schmelzpunkt 241 – 242° C erhält.

Analyse:
Berechnet für $C_{21}H_{19}NO_2 \cdot HBr$: 63,32% C; 5,06% H; 3,52% N; 20,06% Br.
Gefunden: 63,54% C; 5,12% H; 3,46% N; 20,15% Br.

Beispiel 11

Man rührt ein Gemisch aus 1,1 g 10,11-Dihydro-11-oxospiro[dibenz(b,f)oxepin-10,4'-piperidin], der freien Base aus Beispiel 3, 0,63 g $\beta$-Bromäthylalkohol und 1,1 g Natriumbicarbonat in 15 ml Dimethylformamid 16 Stunden bei 80° C. Dann verdünnt man mit Äther und Wasser. Die Ätherlösung wird mit einem Überschuß 2n-Salzsäure extrahiert. Den sauren Extrakt macht man mit Kaliumcarbonat basisch, was ein schweres Öl liefert. Dieses wird in Äther gelöst und die ätherische Lösung getrocknet und zur Trockne eingeengt, wobei ein farbloses Öl verbleibt, welches in Äther in sein Bromwasserstoffsäuresalz überführt wird. Dieses wird aus Methylalkohol/Aceton/Äther umkristallisiert, wobei man 10,11-Dihydro-1'-($\beta$-hydroxyäthyl)-11-oxospiro[dibenz(b,f)oxepin-10,4'-piperidin] als farblose Prismen vom Schmelzpunkt 263 – 266° C erhält.

Analyse:
Berechnet für $C_{20}H_{21}NO_3 \cdot HBr$: 59,41% C; 5,48% H; 3,46% N; 19,76% Br.
Gefunden: 59,18% C; 5,63% H; 3,29% N; 19,70% Br.

Beispiel 12

Man rührt ein Gemisch aus 2,0 g 10,11-Dihydro-11-oxospiro[dibenz(b,f)oxepin-10,4'-piperidin], der freien Base aus Beispiel 3, 0,74 g Chlormethylcyclopropan, 1,8 g Natriumbicarbonat und 1,8 g Kaliumjodid in 15 ml Dimethylformamid 16 Stunden bei 70 – 80° C. Man läßt die entstandene Suspension abkühlen und verdünnt dann mit Äther und Wasser. Die organische Phase wird nacheinander getrocknet, filtriert und im Vakuum eingeengt, wobei ein viskoses Öl verbleibt. Dieses löst man in Chloroform und läßt die Chloroformlösung mit Äther als Eluiermittel durch eine Aluminiumoxydsäule laufen. Das gereinigte Öl wird in Äther gelöst und in sein Bromwasserstoffsäuresalz überführt, welches aus Methylalkohol/Äther umkristallisiert wird, wobei man 1'-Cyclopropylmethyl-10,11-dihydro-11-oxospiro[dibenz(b,f)oxepin-10,4'-piperidin]-hydrobromid als rhombische Kristalle vom Schmelzpunkt 258 – 259° C (Zersetzung) erhält.

Analyse:
Berechnet für $C_{22}H_{23}NO_2 \cdot HBr$: 63,77% C; 5,84% H; 3,38% N; 19,31% Br.
Gefunden: 63,52% C; 5,81% H; 3,34% N; 19,38% Br.

Beispiele 13 und 14

Man behandelt Proben von 10,11-Dihydro-11-oxospiro[dibenz(b,f)oxepin-10,4'-piperidin], der freien Base aus Beispiel 3, nach der Arbeitsweise von Beispiel 12 getrennt mit Phenoxypropylbromid bzw. 2-Bromäthylbenzol, wobei man, Beispiel 13, 10,11-Dihydro-11-oxo-1'-(3-phenoxypropyl)-spiro[dibenz(b,f)oxepin-10,4'-piperidin]-hydrobromid (aus Äthylalkohol/Äther) als farblose Prismen vom Schmelzpunkt 213 – 215° C (Zersetzung) bzw., Beispiel 14, 10,11-Dihydro-11-oxo-1'-phenäthylspiro[di-

benz(b,f)oxepin-10,4'-piperidin]-hydrobromid (aus Aceton/Äther) als farblose Prismen vom Schmelzpunkt >300° C erhält.

Analyse:

Beispiel 13:

Berechnet für $C_{27}H_{27}NO_3 \cdot HBr$: 65,59% C; 5,70% H; 2,83% N.
Gefunden: 65,37% C; 5,73% H; 2,80% N.

Beispiel 14:

Berechnet für $C_{26}H_{25}NO_2 \cdot HBr$: 67,20% C; 5,67% H; 3,30% N; 17,28% Br.
Gefunden: 66,69% C; 5,56% H; 3,17% N; 17,04% Br.

## Beispiel 15

Man rührt ein Gemisch aus 1,3 g 10,11-Dihydro-11-oxospiro[dibenz(b,f)oxepin-10,4'-piperidin], der freien Base aus Beispiel 3, 1,4 g α-Chlor-4-fluorbutyrophenon-äthylenglykolketal, 1,0 g Natriumbicarbonat und 1,0 g Kaliumjodid in 15 ml Dimethylformamid 16 Stunden bei 80° C. Anschließend wird das Reaktionsgemisch filtriert und das Filtrat eingeengt, wobei die Verbindung 10,11-Dihydro-1'-[4-fluorbutyrophenon]-11-oxospiro[dibenz(b,f)oxepin-10,4'-piperidin]-äthylenglykolketal als öliger Rückstand verbleibt. Dieser wird in durch Vermischen von 16 ml absolutem Äthylalkohol mit 6 ml 3n-Salzsäure hergestellter äthanolischer Salzsäure gelöst und 16 Stunden bei Raumtemperatur gerührt. Die saure Lösung macht man mit 40%iger Natronlauge alkalisch, wobei ein Amin frei wird, das mit Äther extrahiert wird. Man trocknet die vereinigten Ätherextrakte und engt dann zur Trockne ein. Den Rückstand läßt man mit Äther als Eluiermittel durch eine Aluminiumoxydsäule laufen, was ein farbloses Öl liefert. Dieses wird in Äther in sein Bromwasserstoffsäuresalz überführt, welches aus Methylalkohol/Äther umkristallisiert wird, wobei man 10,11-Dihydro-1'-[3-(4-fluorbenzoyl)-propyl]-11-oxospiro[dibenz(b,f)oxepin-10,4'-piperidin]-hydrobromid als farblose Prismen vom Schmelzpunkt 260–262° C erhält.

Analyse:

Berechnet für $C_{23}H_{26}FNO_3 \cdot HBr$: 64,13% C; 5,29% H; 2,67% N; 15,24% Br.
Gefunden: 64,18% C; 5,28% H; 2,38% N; 15,49% Br.

## Beispiel 16

Man rührt ein Gemisch aus 2,5 g 2-Chlor-10,11-dihydro-11-oxospiro[dibenz(b,f)oxepin-10,4'-piperidin], der freien Base aus Beispiel 5, 2,5 g Natriumbicarbonat, 2,0 g Kaliumjodid und 0,8 g Chlormethylcyclopropan in 30 ml Dimethylformamid 16 Stunden bei 80° C. Anschließend kühlt man im Eisbad und verdünnt dann mit 90 ml Wasser. Die wäßrige Phase wird zweimal mit je 40 ml Äther extrahiert, mit 58%igem Ammoniumhydroxyd auf pH 9 gestellt und erneut zweimal mit je 40 ml Äther extrahiert. Die vereinigten Ätherextrakte wäscht man nacheinander zweimal mit je 40 ml Wasser und einmal mit 20 ml gesättigter Kochsalzlösung und bringt zur Trockne, was ein Öl liefert. Dieses läßt man mit Äther als Eluiermittel durch eine Aluminiumoxydsäule laufen, wobei ein gereinigtes Öl entsteht, welches man in Äther löst und in sein Bromwasserstoffsäuresalz überführt, das aus Methylalkohol/Aceton/Äther umkristallisiert wird, wobei man 2-Chlor-1'-cyclopropylmethyl-10,11-dihydro-11-oxo[dibenz(b,f)oxepin-10,4'-piperidin]-hydrobromid als weißes Pulver vom Schmelzpunkt 305+°C (Zersetzung) erhält.

Analyse:

Berechnet für $C_{22}H_{22}ClNO_2 \cdot HBr$: 58,88% C; 5,17% H; 3,12% N; 25,71% Gesamthalogen.
Gefunden: 58,76% C; 5,35% H; 3,05% N; 25,73% Gesamthalogen.

## Beispiel 17

Man behandelt ein Gemisch aus 2,5 g 2-Chlor-10,11-dihydro-11-oxospiro[dibenz(b,f)oxepin-10,4'-piperidin], der freien Base aus Beispiel 5, 2,5 g Natriumbicarbonat, 2,0 g Kaliumjodid und 1,1 g 2-Propinylbromid in 30 ml Dimethylformamid gemäß der Arbeitsweise von Beispiel 16, was ein chromatographiertes gereinigtes Öl ergibt. Dieses wird mit Hexan überschichtet und auf 0° C abgekühlt, wobei sich weiße Kristalle bilden, die aus Benzol umkristallisiert werden, wobei man 2-Chlor-10,11-dihydro-11-oxo-1'-(2-propinyl)-spiro[dibenz(b,f)oxepin-10,4'-piperidin] als kristallinen Feststoff vom Schmelzpunkt 148–150° C erhält.

Analyse:
Berechnet für $C_{21}H_{18}ClNO_2$: 71,69% C; 5,16% H; 3,98% N; 10,18% Cl.
Gefunden: 71,80% C; 5,27% H; 3,88% N; 10,18% Cl.

Beispiel 18

Ein Gemisch aus 0,9 g Allylbromid und Dimethylformamid gibt man portionsweise im Verlauf von 60 Minuten zu einem 65°C warmen Gemisch aus 2,1 g 2-Chlor-10,11-dihydro-11-oxospiro[dibenz(b,f)oxepin-10,4'-piperidin], der freien Base aus Beispiel 5, und 2,1 g Natriumbicarbonat in 15 ml Dimethylformamid. Nach beendeter Zugabe rührt man 16 Stunden bei 60–65°C. Anschließend wird mit 75 ml Wasser verdünnt und das zweiphasige Gemisch mit 40 ml Äther extrahiert. Ein weißer Niederschlag bildet sich an der Grenzfläche. Die wäßrige Schicht und die Grenzflächensubstanz werden dreimal mit je 40 ml Methylenchlorid extrahiert. Die vereinigten organischen Phasen wäscht man nacheinander zweimal mit je 50 ml Wasser und mit 25 ml gesättigter Kochsalzlösung und bringt zur Trockne, was ein Öl liefert. Dieses läßt man mit Äther als Eluiermittel durch eine Aluminiumoxydsäule laufen, was ein gereinigtes Öl ergibt, das in Äther gelöst und in sein Bromwasserstoffsäuresalz überführt wird. Dieses wird aus Methylalkohol umkristallisiert, wobei man 1'-Allyl-2-chlor-10,11-dihydro-11-oxospiro[dibenz(b,f)oxepin-10,4'-piperidin]-hydrobromid als weißen kristallinen Feststoff vom Schmelzpunkt 292–295°C (Zersetzung) erhält.

Analyse:
Berechnet für $C_{21}H_{20}ClNO_2 \cdot HBr$: 58,01% C; 4,87% H; 3,22% N; 26,53% Gesamthalogen.
Gefunden: 58,02% C; 4,93% H; 3,16% N; 26,45% Gesamthalogen.

Beispiel 19

Ein Gemisch aus 1,2 g Äthyljodid und 10 ml Dimethylformamid gibt man portionsweise im Verlauf von 30 Minuten zu einem 80°C warmen Gemisch aus 2,1 g 2-Chlor-10,11-dihydro-11-oxospiro[dibenz(b,f)oxepin-10,4'-piperidin], der freien Base aus Beispiel 5, und 2,5 g $NaHCO_3$ in 15 ml Dimethylformamid. Nach beendeter Zugabe kühlt man das Reaktionsgemisch auf 0°C, verdünnt dann mit 75 ml Wasser und extrahiert danach mit 40 ml Äther. Beide Phasen werden durch Papier hindurch filtriert, und der Filterkuchen wird in 75 ml Chloroform gelöst. Man wäscht die Chloroformlösung mit Wasser und bringt dann zur Trockne, wobei ein Öl verbleibt. Die wäßrige Phase wird viermal mit je 40 ml Äther extrahiert. Die vereinigten Ätherextrakte wäscht man nacheinander zweimal mit je 40 ml Wasser und dann einmal mit 20 ml gesättigter Kochsalzlösung, was ein Öl liefert. Dieses und das obige Öl läßt man mit Äther als Eluiermittel durch eine Aluminiumoxydsäule laufen, was ein gereinigtes Öl ergibt. Dieses wird in Äther gelöst und in sein Bromwasserstoffsäuresalz überführt, ein weißes Pulver, welches aus Methylalkohol umkristallisiert wird, wobei man 2-Chlor-1'-äthyl-10,11-dihydro-11-oxospiro[dibenz(b,f)oxepin-10,4'-piperidin]-hydrobromid als klare rhombische Plättchen vom Schmelzpunkt 303–304°C erhält.

Analyse:
Berechnet für $C_{20}H_{20}ClNO_2 \cdot HBr$: 56,82% C; 5,01% H; 3,31% N; 27,29% Gesamthalogen.
Gefunden: 56,94% C; 5,16% H; 3,28% N; 27,07% Gesamthalogen.

Beispiel 20

Man rührt ein Gemisch aus 2,1 g 2-Chlor-10,11-dihydro-11-oxospiro[dibenz(b,f)oxepin-10,4'-piperidin], der freien Base aus Beispiel 5, und 2,1 g Natriumbicarbonat in 15 ml Dimethylformamid bei 85°C und gibt dann im Verlauf von 30 Minuten portionsweise ein Gemisch aus 1,3 g Jodpropan und 10 ml Dimethylformamid dazu. Nach beendeter Zugabe wird der Ansatz 16 Stunden bei 80–85°C gerührt und dann 48 Stunden bei Raumtemperatur stehengelassen. Danach verdünnt man mit 75 ml Wasser und extrahiert dreimal mit je 40 ml Äther. Die vereinigten Ätherextrakte werden nacheinander zweimal mit je 30 ml Wasser und mit 20 ml gesättigter Kochsalzlösung gewaschen und zur Trockne gebracht, was ein Öl liefert. Dieses löst man in einem Gemisch aus Äthylalkohol und 3n-Salzsäure (2 : 1). Die saure Lösung wird mit 58%iger Ammoniumhydroxydlösung auf pH 9 basisch gemacht und dann viermal mit je 40 ml Äther extrahiert, was ein Öl liefert. Dieses läßt man mit Äther als Eluiermittel durch eine Aluminiumoxydsäule laufen, wobei ein gereinigtes Öl entsteht. Dieses löst man in Äther und überführt es in sein Bromwasserstoffsäuresalz. Die ätherische Lösung versetzt man mit Methylalkohol, entfernt den Äther durch Erhitzen und gibt dann Aceton dazu, wobei man 2-Chlor-10,11-dihydro-11-oxo-1'-propylspiro[dibenz(b,f)oxepin-10,4'-piperidin]-hydrobromid als weißes Pulver vom Schmelzpunkt 315–316°C erhält.

Analyse:
Berechnet für $C_{21}H_{22}ClNO_2 \cdot HBr$: 57,75% C; 5,31% H; 3,21% N; 26,42% Gesamthalogen.
Gefunden: 58,02% C; 5,38% H; 3,29% N; 26,36% Gesamthalogen.

Beispiel 21

Ein Gemisch aus 1,0 g 1-Brom-3-methyl-2-buten und 10 ml Dimethylformamid gibt man portionsweise im Verlauf von 20 Minuten unter Rühren zu einem 80°C warmen Gemisch aus 1,9 g 2-Chlor-10,11-dihydro-11-oxospiro[dibenz(b,f)oxepin-10,4'-piperidin], der freien Base aus Beispiel 5, und 1,9 g Natriumbicarbonat in 15 ml Dimethylformamid. Nach beendeter Zugabe rührt man 16 Stunden bei 80°C. Danach wird mit 75 ml Wasser verdünnt und das verdünnte Gemisch dreimal mit je 40 ml Äther extrahiert. Die vereinigten Ätherextrakte wäscht man nacheinander zweimal mit je 30 ml Wasser und dann mit 20 ml gesättigter Kochsalzlösung und bringt zur Trockne, was ein Öl liefert. Dieses wird auf vier 20 × 20 cm großen präparativen Silikagelschichtplatten F 254 in einem 10%igen Methylalkohol/Benzolgemisch chromatographiert. Eine Bande (Rf 0,25 – 0,61) wird eluiert und ergibt ein Öl. Dieses löst man in Äther und überführt es in sein Bromwasserstoffsäuresalz. Dieses wird aus Methylalkohol/Aceton/Äther umkristallisiert, wobei man 2-Chlor-10,11-dihydro-1'-(2-methyl-2-butenyl)-11-oxospiro[dibenz(b,f)oxepin-10,4'-piperidin]-hydrobromid als weißes Pulver vom Schmelzpunkt 266,5 – 267,5°C (Zersetzung) erhält.

Analyse:
Berechnet für $C_{23}H_{24}ClNO_2 \cdot HBr$: 59,69% C; 5,45% H; 3,03% N; 24,93% Gesamthalogen.
Gefunden: 59,62% C; 5,36% H; 2,99% N; 24,66% Gesamthalogen.

Beispiel 22

Eine Lösung von 0,93 g 2-Bromäthylalkohol in 10 ml Dimethylformamid gibt man portionsweise im Verlauf von 30 Minuten unter Rühren zu einem 80°C warmen Gemisch aus 2,1 g 2-Chlor-10,11-dihydro-11-oxospiro[dibenz(b,f)oxepin-10,4'-piperidin], der freien Base aus Beispiel 5, 2,1 g Natriumbicarbonat und 1,7 g Kaliumjodid in 15 ml Dimethylformamid. Nach beendeter Zugabe rührt man 16 Stunden bei 80 – 85°C. Danach wird mit 75 ml Wasser verdünnt und das verdünnte Gemisch dreimal mit je 40 ml Äther extrahiert. Die vereinigten Ätherextrakte wäscht man nacheinander zweimal mit je 30 ml Wasser und extrahiert zweimal mit je 36 ml 2n-Salzsäure. Das vereinigte saure Gemisch wird abgekühlt und dann mit 58%igem Ammoniumhydroxyd auf pH 9 basisch gemacht. Das basische Gemisch extrahiert man dreimal mit je 40 ml Äther, wäscht anschließend die vereinigten Ätherextrakte einmal mit 40 ml Wasser und bringt zur Trockne, was ein Öl liefert. Dieses läßt man mit Methylalkohol/Äther (1 : 1) als Eluiermittel durch eine Aluminiumoxydsäule laufen, was einen Feststoff ergibt. Dieser wird in Äther gelöst und filtriert, wonach man einen glasigen Feststoff erhält, der in 15%igem Aceton/Äthergemisch gelöst und in sein Bromwasserstoffsäuresalz überführt wird, welches aus Methylalkohol/Aceton/Äther umkristallisiert wird, wobei man 2-Chlor-10,11-dihydro-1'-(2-hydroxyäthyl)-11-oxospiro[dibenz(b,f)oxepin-10,4'-piperidin]-hydrobromid als weißes Pulver vom Schmelzpunkt 240 – 242°C erhält.

Analyse:
Berechnet für $C_{20}H_{20}ClNO_3 \cdot HBr$: 54,75% C; 4,82% H; 3,19% N; 26,29% Gesamthalogen.
Gefunden: 54,89% C; 5,07% H; 3,10% N; 26,18% Gesamthalogen.

Beispiel 23

Man rührt ein Gemisch aus 2,1 g 2-Chlor-10,11-dihydro-11-oxospiro[dibenz(b,f)oxepin-10,4'-piperidin], der freien Base aus Beispiel 5, 2,1 g Natriumbicarbonat, 1,7 g Kaliumjodid und 1,4 g 2-Bromäthylbenzol in 25 ml Dimethylformamid 18 Stunden bei 50 – 65°C. Danach wird mit 75 ml Wasser verdünnt und das verdünnte Gemisch dreimal mit je 40 ml Äther extrahiert. Die vereinigten Ätherextrakte wäscht man nacheinander zweimal mit je 30 ml Wasser und einmal mit 20 ml gesättigter Kochsalzlösung und bringt zur Trockne, wobei ein Öl verbleibt. Dieses läßt man mit Äther als Eluiermittel durch eine Aluminiumoxydsäule laufen, was ein gereinigtes Öl ergibt. Dieses wird mit Hexan behandelt und das Hexangemisch abgekühlt und filtriert, was eine weiße kristalline Substanz ergibt, die aus Aceton umkristallisiert wird, wobei man 2-Chlor-10,11-dihydro-11-oxo-1'-($\beta$-phenyläthyl)-spiro[dibenz(b,f)oxepin-10,4'-piperidin] als weißen Feststoff vom Schmelzpunkt 136,5 – 138°C erhält.

Analyse:
Berechnet für $C_{26}H_{24}ClNO_2$: 74,72% C; 5,79% H; 3,35% N; 8,48% Cl.
Gefunden: 74,97% C; 5,92% H; 3,44% N; 8,38% Cl.

Beispiel 24

Im Verlauf von 20 Minuten gibt man eine Lösung von 1,6 g 3-Phenoxypropylbromid in 10 ml Dimethylformamid portionsweise zu einem 80° C warmen Gemisch aus 2,1 g 2-Chlor-10,11-dihydro-11-oxospiro[dibenz(b,f)oxepin-10,4'-piperidin], der freien Base aus Beispiel 5, 2,1 g Natriumbicarbonat und 1,7 g Kaliumjodid in 15 ml Dimethylformamid. Nach beendeter Zugabe rührt man 16 Stunden bei 80—85° C. Danach wird im Eisbad abgekühlt und anschließend mit 75 ml Wasser verdünnt. Das verdünnte Gemisch extrahiert man mit 40 ml Äther und läßt beide Phasen (die organische und die wäßrige) durch Filterpapier laufen. Die filtrierte wäßrige Phase wird dreimal mit je 40 ml Äther extrahiert und die vereinigten Ätherextrakte nacheinander zweimal mit je 40 ml Wasser und einmal mit 20 ml gesättigter Kochsalzlösung gewaschen und zur Trockne gebracht, wobei ein Öl verbleibt. Das rohe Öl wird gereinigt, indem man es mit Äther als Eluiermittel durch eine Aluminiumoxydsäule laufen läßt. Das gereinigte Öl wird in Äther gelöst und die ätherische Lösung stehengelassen, wobei sich ein weißer Niederschlag bildet. Der Niederschlag wird durch Filtrieren gewonnen und dann mit Äther gewaschen, wobei man 2-Chlor-10,11-dihydro-11-oxo-1'-(3-phenoxypropyl)-spiro[dibenz(b,f)oxepin-10,4'-piperidin] als weiße kristalline Substanz vom Schmelzpunkt 105—107° C erhält.

Analyse:

| | | | | |
|---|---|---|---|---|
| Berechnet für $C_{27}H_{26}ClNO_3$: | 72,39% C; | 5,85% H; | 3,13% N; | 7,91% Cl. |
| Gefunden: | 72,33% C; | 5,94% H; | 3,13% N; | 8,02% Cl. |

Beispiel 25

Ein Gemisch aus 1,8 g 4-Chlor-p-fluorbutyrophenon-äthylenketal und 10 ml Dimethylformamid gibt man im Verlauf von 30 Minuten portionsweise zu einem 85°C warmen Gemisch aus 2,1 g 2-Chlor-10,11-dihydro-11-oxospiro[dibenz(b,f)oxepin-10,4'-piperidin], der freien Base aus Beispiel 5, 1,7 g Kaliumjodid und 2,1 g Natriumbicarbonat in 15 ml Dimethylformamid. Nach beendeter Zugabe rührt man 16 Stunden bei 80—85° C und läßt dann 48 Stunden stehen. Danach wird mit 75 ml Wasser verdünnt und das verdünnte Gemisch dreimal mit je 40 ml Äther extrahiert. Die vereinigten Ätherextrakte wäscht man zweimal mit je 30 ml Wasser und dampft dann am Rotationsverdampfer ein, wobei man die Verbindung 2-Chlor-1'-[4-fluorbutyrophenon]-10,11-dihydro-11-oxospiro[dibenz(b,f)oxepin-10,4'-piperidin]-äthylenglykolketal als glasigen Rückstand erhält. Der Rückstand wird in einem Gemisch aus 55 ml Äthylalkohol und 21 ml 3n-Salzsäure gelöst. Die saure Lösung rührt man 19 Stunden bei Raumtemperatur. Das Gemisch wird unter gutem Rühren im Eisbad gekühlt und dann mit 58%igem Ammoniumhydroxyd auf pH 9 basisch gemacht. Das basische Gemisch extrahiert man dreimal mit je 40 ml Äther, wäscht die vereinigten Ätherextrakte nacheinander zweimal mit je 30 ml Wasser und einmal mit 20 ml gesättigter Kochsalzlösung, trocknet und dampft zur Trockne ein, was ein Öl ergibt. Dieses wird gereinigt, indem man es mit Äther als Eluiermittel durch eine Aluminiumoxydsäule laufen läßt. Das gereinigte Öl wird in Äther gelöst und als sein Bromwasserstoffsäuresalz gefällt. Das Salz wird mit Äther gewaschen und dann aus Aceton/Äther umkristallisiert, wobei man 2-Chlor-1'-[3-(4-fluorbenzoyl)-propyl]-10,11-dihydro-11-oxospiro[dibenz(b,f)oxepin-10,4'-piperidin]-hydrobromid als weißes Pulver vom Schmelzpunkt 220—221° C erhält.

Analyse:

| | | | | |
|---|---|---|---|---|
| Berechnet für $C_{28}H_{25}ClFNO_3 \cdot HBr$: | 60,17% C; | 4,69% H; | 2,51% N; | 20,64% Gesamthalogen. |
| Gefunden: | 60,10% C; | 4,66% H; | 2,52% N; | 20,68% Gesamthalogen. |

Beispiel 26

a) Man hält ein Gemisch aus 147 g Jodbenzoesäure und 45,5 g Kaliumcarbonat in 57 ml Nitrobenzol 40 Minuten unter Rühren bei 160° C. Danach werden weitere 46,5 g Kaliumcarbonat und anschließend 73,1 g 4-Fluorphenol, nochmals 46,5 g Kaliumcarbonat und 0,35 g Kupferpulver zugesetzt. Das Reaktionsgemisch wird 45 Minuten in einem Bad bei 160° C gerührt. Der entstandene Feststoff wird isoliert und dann auf 0° C gekühlt, bevor man ihn mit 100 ml Wasser und 200 ml 6n-Salzsäure vermischt. Dann verdünnt man das wäßrige Gemisch auf 1 Liter Gesamtvolumen und rührt dann mit 450 ml Chloroform. Der entstandene weiße Feststoff wird isoliert und nacheinander mit Chloroform und Wasser gewaschen. Der Feststoff wird in heißem Aceton aufgelöst und die Acetonlösung zuerst filtriert und dann abgekühlt, was ein weißes kristallines Produkt liefert. Das Produkt kristallisiert man aus Aceton um und überführt es dann mit Kaliumcarbonat in ein Salz. Das Salz wird in Wasser gelöst und die wäßrige Lösung mit 1n-Salzsäure angesäuert, wobei man 2-(4-Fluorphenoxy)-benzoesäure als weiße Kristalle vom Schmelzpunkt 143—146° C erhält.

b) Unter Stickstoff bei Raumtemperatur tropft man 36,1 ml 70%iges Natrium-(2-methoxyäthyl)-aluminiumhydrid in Benzol im Verlauf einer Stunde unter Rühren zu einem Gemisch aus 15 g

2-(4-Fluorphenoxy)-benzoesäure und 150 ml Benzol. Nach beendeter Zugabe rührt man noch 30 Minuten weiter und läßt das Reaktionsgemisch dann 4 Tage lang stehen. Danach kühlt man auf 0°C ab und versetzt dann unter Rühren mit 100 ml 10%iger Natronlauge. Nach dieser Zugabe scheiden sich die Schichten, und die wäßrige Schicht wird zweimal mit je 100 ml Benzol extrahiert. Die vereinigten Benzollösungen wäscht man nacheinander zweimal mit je 100 ml Wasser und einmal mit 60 ml gesättigter Kochsalzlösung und bringt zur Trockne, was ein klares gelbes Öl liefert. Das Öl wird bei 118,5 – 121,5°C und 0,07 Torr destilliert, wobei man 2-(4-Fluorphenoxy)-benzylalkohol als klare farblose Flüssigkeit erhält.

c) Unter Rühren bei Raumtemperatur tropft man 8,5 g Thionylchlorid im Verlauf von 10 Minuten zu einem Gemisch aus 13,0 g 2-(4-Fluorphenoxy)-benzylalkohol und 80 ml Benzol. Nach beendeter Zugabe rührt man 16 Stunden und gießt das Reaktionsgemisch dann vorsichtig auf 350 ml zerkleinertes Eis, das 8 ml Natriumbicarbonat enthält. Man rührt, bis das Eis schmilzt. Die Schichten scheiden sich, und die Benzolschicht wird nacheinander zweimal mit je 40 ml halbgesättigter Natriumbicarbonatlösung, zweimal mit je 40 ml Wasser und einmal mit 25 ml gesättigter Kochsalzlösung gewaschen, getrocknet und am Rotationsverdampfer eingedampft, was eine klare Flüssigkeit liefert. Diese wird bei 106 – 109°C und 0.16 Torr destilliert, wobei man 2-(4-Fluorphenoxy)-benzylchlorid als klare farblose Flüssigkeit erhält.

d) Im Verlauf von 30 Minuten gibt man 12,0 g 2-(4-Fluorphenoxy)-benzylchlorid portionsweise zu einem Gemisch aus 2,9 g Natriumcyanid und 120 ml Dimethylsulfoxyd. Nach beendeter Zugabe rührt man 26 Stunden und gießt dann auf 150 – 200 ml zerkleinertes Eis. Man extrahiert viermal mit je 70 ml Äther und wäscht die vereinigten Ätherextrakte nacheinander viermal mit je 50 ml Wasser und einmal mit 25 ml gesättigter Kochsalzlösung und bringt zur Trockne, was ein klares Öl liefert. Dieses wird bei 128,5 – 130°C und 0,1 Torr destilliert, wobei man 2-(4-Fluorphenoxy)-benzylcyanid erhält.

e) Unter Stickstoff gibt man 2,3 g 99%iges Natriumhydrid langsam zu einem Gemisch aus 4,3 g 2-(4-Fluorphenoxy)-benzylcyanid und 30 ml Dimethylsulfoxyd. Nach beendeter Zugabe rührt man fünf Minuten weiter und gibt dann im Verlauf von 40 Minuten ein Gemisch aus 3,9 g 95%igem Mechloräthaminhydrochlorid und 20 ml Dimethylsulfoxyd portionsweise dazu. Nach dieser Zugabe rührt man 1 Stunde in einem Heißwasserbad bei 70°C und läßt dann 16 Stunden bei Raumtemperatur stehen. Das abgekühlte Reaktionsgemisch gießt man auf 150 ml zerkleinertes Eis und extrahiert das verdünnte Gemisch dreimal mit je 60 ml Äther. Die vereinigten Ätherextrakte werden mit 25 ml gesättigter Kochsalzlösung gewaschen und zur Trockne gebracht, was einen gelben Feststoff liefert. Dieser wird aus Cyclohexan umkristallisiert, wobei man 4-Cyan-4-[2-(4-fluorphenoxy)-phenyl]-1-methylpiperidin als weißen Feststoff vom Schmelzpunkt 106 – 107,5°C erhält.

Analyse:

| | | | | |
|---|---|---|---|---|
| Berechnet für $C_{19}H_{19}FN_2O$: | 73,52% C; | 6,16% H; | 9,03% N; | 6,12% F. |
| Gefunden: | 73,47% C; | 6,04% H; | 8,99% N; | 6,23% F. |

f) Man rührt ein Gemisch aus 4,4 g 4-Cyan-4-[2-(4-fluorphenoxy)-phenyl]-1-methylpiperidin und 95 ml 48%iger Bromwasserstoffsäure 16 Stunden unter Rückfluß. Danach verdünnt man mit 300 ml Wasser und dampft bei 80°C am Rotationsverdampfer ein, was einen hellbraunen Feststoff liefert. Dieser wird mit 300 ml Wasser umgeschwenkt und durch Erhöhung des pH auf 9 mit konzentriertem Ammoniumhydroxyd aufgelöst. Die Lösung läßt man 96 Stunden stehen. Der pH wird langsam auf 4 erniedrigt und dann mit konzentriertem Ammoniumhydroxyd wieder auf 9 gebracht. Das Volumen wird durch Rotationsverdampfung bei 80°C verringert, bis ein Niederschlag in Erscheinung tritt. Anschließend wird das Reaktionsgemisch gekühlt und der Niederschlag durch Filtrieren gesammelt und dann mit Wasser gewaschen. Nacheinander wird der Niederschlag mit Aceton gewaschen, mit 1 ml mit 20 ml Wasser verdünnter 48%iger Bromwasserstoffsäure behandelt und auf Bio-Rad A 650 W-X8-Kationenaustauscherharz aufgebracht und mit 5,8 – 8,7%igem Ammoniumhydroxyd eluiert, wobei man 4-[2-(4-Fluorphenoxy)-phenyl]-1-methylpiperidin-4-carbonsäure als weißes Pulver vom Schmelzpunkt 284 – 286°C erhält.

Analyse:

| | | | | |
|---|---|---|---|---|
| Berechnet für $C_{19}H_{20}FNO_3$: | 69,28% C; | 6,12% H; | 4,25% N; | 5,77% F. |
| Gefunden: | 69,04% C; | 5,98% H; | 4,34% N; | 5,63% F. |

g) Man rührt ein Gemisch aus 2,5 g 4-[2-(4-Fluorphenoxy)-phenyl]-1-methylpiperidin-4-carbonsäure und 24 ml Polyphosphorsäure 2 Stunden unter Stickstoff in einem Bad bei 100 – 115°C. Danach kühlt man im Eisbad und verdünnt dann mit 50 ml Wasser und 15 ml Eis. Das gekühlte verdünnte Gemisch wird mit 58%igem Ammoniumhydroxyd auf pH 9 – 10 basisch gemacht und dreimal mit je 50 ml Äther/Methylenchlorid (2 : 1) extrahiert. Man vereinigt die Extrakte und wäscht nacheinander einmal mit 75 ml 10%iger Natronlauge, dann mit 75 ml Wasser und schließlich mit 20 ml gesättigter Kochsalzlösung und trocknet dann. Die getrocknete Lösung wird zur Trockne eingeengt, wobei ein Öl verbleibt, welches man über eine Silikagelsäule mit Methylalkohol/Methylenchlorid (1 : 9) als Eluiermittel chromatographiert, was ein gereinigtes Öl ergibt. Dieses wird in Äther in sein

Bromwasserstoffsäuresalz überführt. Das Salz wird mehrmals aus Methylalkohol/Äther umkristallisiert, wobei man 2-Fluor-10,11-dihydro-1'-methyl-11-oxospiro[dibenz(b,f)oxepin-10,4'-piperidin]-hydrobromid als weiße Kristalle vom Schmelzpunkt 294,0 – 295,5° C erhält.

Analyse:
Berechnet für $C_{19}H_{18}FNO_2 \cdot HBr$: 58,17% C; 4,88% H; 3,57% N; 4,84% F.
Gefunden: 58,08% C; 4,79% H; 3,59% N; 4,71% F.

Beispiel 27

Im Verlauf von 15 Minuten gibt man ein Gemisch aus 3,0 g 2-Fluor-10,11-dihydro-1'-methyl-11-oxospiro[dibenz(b,f)oxepin-10,4'-piperidin], der freien Base aus Beispiel 26, und 10 ml Methylenchlorid portionsweise zu einem Gemisch aus 1,3 g Bromcyan und 6,4 g Kaliumcarbonat in 17 ml Methylenchlorid. Nach beendeter Zugabe rührt man 2 Stunden unter Rückfluß und läßt dann auf Raumtemperatur abkühlen. Anschließend wird das Gemisch filtriert und dann zur Trockne eingedampft, und der Rückstand wird in Methylalkohol aufgenommen. Die alkoholische Lösung wird zum Rückfluß erhitzt und mit Aceton und Äther versetzt, was einen Niederschlag erzeugt. Man filtriert und dampft die überstehende Flüssigkeit ein, wobei ein glasiger Feststoff verbleibt, den man über eine Silikagelsäule mit Äther/Methylenchlorid (1 : 1) als Eluiermittel chromatographiert, um ein reineres Produkt zu erhalten. Dieses wird zunächst in Aceton gelöst und dann mit Äther und Pentan verdünnt und schließlich mehrmals aus Aceton/Äther/Pentan umkristallisiert, wobei man 1'-Cyan-2-fluor-10,11-dihydro-11-oxospiro[dibenz(b,f)oxepin-10,4'-piperidin] als weißes kristallines Pulver vom Schmelzpunkt 105 – 106° C erhält.

Analyse:
Berechnet für $C_{19}H_{15}FN_2O_2$: 70,79% C; 4,69% H; 8,69% N; 5,89% F.
Gefunden: 70,59% C; 4,81% H; 8,63% N; 5,97% F.

Beispiel 28

Man rührt ein Gemisch aus 1,8 g 1'-Cyan-2-fluor-10,11-dihydro-11-oxospiro[dibenz(b,f)oxepin-10,4'-piperidin], Beispiel 27, 11 ml Essigsäure und 21 ml 3n-Salzsäure 20 Stunden in einem Bad bei 125 – 130° C. Anschließend wird mit 25 ml Wasser verdünnt und am Rotationsverdampfer zur Trockne eingedampft. Der Rückstand wird in 25 ml Wasser aufgenommen und das Wasser am Rotationsverdampfer entfernt, wobei ein weißer Feststoff verbleibt. Diesen nimmt man in 25 ml Wasser auf und kühlt das wäßrige Gemisch auf 0° C. Der Niederschlag wird durch Filtrieren gewonnen und dann nacheinander mit Wasser und Äther gewaschen, bevor er zur Trockne gebracht wird. Das Produkt wird mehrmals aus Methylalkohol/Aceton/Äther umkristallisiert, wobei man 2-Fluor-10,11-dihydro-11-oxospiro[dibenz(b,f)oxepin-10,4'-piperidin]-hydrochlorid als weißen körnigen Feststoff vom Schmelzpunkt 297 – 299° C erhält.

Analyse:
Berechnet für $C_{18}H_{16}FNO_2 \cdot HCl$: 64,77% C; 5,13% H; 4,20% N; 5,69% F.
Gefunden: 64,50% C; 5,11% H; 4,10% N; 5,71% F.

Beispiel 29

a) Im Verlauf von 15 Minuten gibt man ein Gemisch aus 19,8 g 4-Cyan-4-[2-(4-fluorphenoxy)-phenyl]-1-methylpiperidin, Beispiel 26(c), und 200 ml Chloroform portionsweise zu einem Gemisch aus 33,4 g 97%igem Bromcyan und 130 g Kaliumcarbonat in 250 ml Chloroform. Nach beendeter Zugabe wird das Gemisch nacheinander 16 Stunden zum Rückfluß erhitzt, abgekühlt, filtriert, dreimal mit je 300 ml Wasser und einmal mit 50 ml gesättigter Kochsalzlösung gewaschen und zur Trockne gebracht, was ein klares schwachgelbes, beim Stehen kristallisierendes Öl ergibt. Der Feststoff wird in Benzol aufgelöst und die Benzollösung nach Zusatz von Aktivkohlepulver aufgekocht. Das Gemisch wird über Celite filtriert, das Volumen dann verringert und wieder mit Hexan verdünnt, wobei man 1,4-Dicyan-4-[2-(4-fluorphenoxy)-phenyl]-piperidin als weißes kristallines Pulver vom Schmelzpunkt 107,5 – 109,5° C erhält.

b) Man rührt ein Gemisch aus 12,0 g 1,4-Dicyan-4-[2-(4-fluorphenoxy)-phenyl]-piperidin und 240 ml 48%iger Bromwasserstoffsäure 40 Stunden in einem Bad bei 150° C, versetzt dann mit 120 ml Eisessig und rührt noch 30 Stunden bei derselben Temperatur weiter. Danach wird die Lösung 64 Stunden stehengelassen und der Überschuß dann abdestilliert, wobei ein breiiger Rückstand verbleibt. Dieser wird in 50 ml Wasser aufgenommen, und das wäßrige Gemisch wird am Rotationsverdampfer

eingedampft, wobei ein Feststoff verbleibt, welcher beim Waschen mit Wasser und dann Äther zu einem hellbraungrauen Pulver wird. Dieses wird in 250 ml Methylalkohol aufgelöst und die alkoholische Lösung nach Zusatz von Aktivkohle aufgekocht; nach Filtration und Eindampfen erhält man ein weißes kristallines Pulver. Dieses wird dreimal aus Methylalkohol/Äther und einmal aus Äthylalkohol/Wasser umkristallisiert und getrocknet, wobei man 4-[2-(4-Fluorphenoxy)-phenyl]-piperidin-4-carbonsäurehydrobromid als Produkt mit einem Schmelzbereich von 180–250°C (Zersetzung) erhält.

c) Man rührt ein Gemisch aus 0,7 g 4-[2-(4-Fluorphenoxy)-phenyl]-piperidin-4-carbonsäure und 0,83 ml Essigsäureanhydrid in 4,2 ml Pyridin 4 Stunden unter Rückfluß. Anschließend entfernt man überschüssiges Pyridin am Rotationsverdampfer und nimmt den Rückstand in 10 ml Wasser auf, worin er mit 10 ml 1n-Salzsäure behandelt wird. Das saure Gemisch extrahiert man dreimal mit je 20 ml Äther/Benzol (1 : 1) und wäscht die vereinigten Extrakte nacheinander zweimal mit je 40 ml Wasser, einmal mit 7 ml gesättiger Kupfersulfatlösung und einmal mit 15 ml gesättigter Kochsalzlösung und bringt zur Trockne, was einen glasigen Feststoff liefert. Dieser wird aus Aceton/Äther (1 : 4) und dann mehrmals aus Aceton umkristallisiert, wobei man 1-Acetyl-4-[2-(4-fluorphenoxy)-phenyl]-piperidin-4-carbonsäure als weißes kristallines Pulver vom Schmelzpunkt 192,5–193,5°C erhält.

d) Man versetzt ein Gemisch aus 0,3 g 1-Acetyl-4-[2-(4-fluorphenoxy)-phenyl]-piperidin-4-carbonsäure und 3 ml Methylenchlorid mit 0,13 ml frisch destilliertem Thionylchlorid. Die entstandene rosafarbene Lösung rührt man 155 Minuten und verdünnt dann mit 7,5 ml Methylenchlorid. Die Reaktionslösung wird tropfenweise zu 0,17 g Aluminiumchlorid gegeben. Anschließend rührt man 15 Minuten und erhitzt dann drei Stunden zum Rückfluß. Das Gemisch wird unter Rückfluß 72 Stunden gerührt und dann nacheinander mit Eis, 25 ml Wasser und 25 ml Chloroform versetzt. Nach Umschütteln scheiden sich die Schichten. Die wäßrige Schicht wird zweimal mit je 25 ml Chloroform extrahiert, und die vereinigten Chloroformlösungen werden nacheinander einmal mit 20 ml 10%iger Natronlauge, zweimal mit je 30 ml Wasser und mit 1 ml gesättigter Kochsalzlösung gewaschen, getrocknet und eingedampft, wobei ein Öl verbleibt. Dieses wird auf 20×20 cm großen präparativen Dickschichtsilikagelplatten mit Aceton/Äther/Methylenchlorid (1 : 1 : 1) chromatographiert. Die Band mit hohem Rf wird verworfen und die Hauptbande zu einem glasigen Feststoff eingeengt. Dieser wird in Benzol gelöst und mit Hexan verdünnt, und nach Entfernung des Lösungsmittels verbleibt ein weißes kristallines Pulver. Dieses wird aus Benzol/Hexan umkristallisiert, wobei man 1'-Acetyl-2-fluor-10,11-dihydro-11-oxospiro[dibenz(b,f)oxepin-10,4'-piperidin] als Produkt vom Schmelzpunkt 143–145°C erhält.

Analyse:

Berechnet für $C_{20}H_{18}FNO_3$: 70,78% C; 5,36% H; 4,13% N.
Gefunden: 70,70% C; 5,15% H; 3,94% N.

Weitere erfindungsgemäße Verbindungen lassen sich im Einklang mit den Arbeitsweisen der obenstehenden Beispiele herstellen.

**Patentansprüche für die Vertragsstaaten: BE, CH, DE, FR, GB, IT, NL, SE**

1. Verbindungen der Formel

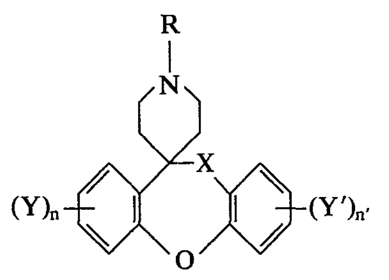

oder deren pharmazeutisch unbedenkliche Säureadditionssalze, worin R für Wasserstoff, $C_1-C_6$-Alkyl, $C_2-C_6$-Alkenyl, $C_2-C_6$-Alkinyl, $C_1-C_6$-Hydroxyalkyl, $C_3-C_7$-Cycloalkyl-$C_1-C_6$-alkyl, gegebenenfalls am Ring substituiertes Phenyl-$C_1-C_6$-alkyl, Phenoxy-$C_1-C_6$-alkyl oder Benzoyl-$C_1-C_6$-alkyl, wobei die Substituenten am Ring Nitro, Amino, Chlor, Fluor, Brom, Methoxy, $C_1-C_6$-Alkyl oder Trifluormethyl sein können, $C_2-C_6$-Alkanoyl, Cyan oder ein Äthylen-glykolketal der Formel

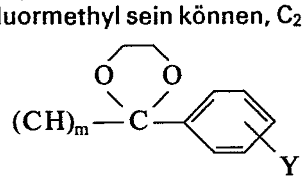

steht, X

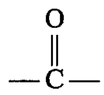 oder 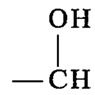

darstellt, wenn R die oben angegebene Bedeutung hat, oder

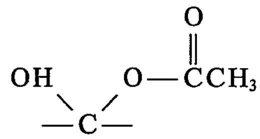

darstellt, wenn R für $C_2-C_6$–Alkanoyl, Methyl oder Cyan steht, Y und Y' gleich oder verschieden sind und je Wasserstoff, Chlor, Fluor, Brom, Methoxy, Methylthio oder Trifluormethyl bedeuten können, n und n' gleich oder verschieden sind und je eine ganze Zahl von 1 bis einschließlich 2 darstellen können und m eine ganze Zahl von 1 bis einschließlich 4 ist.

2. Verbindungen nach Anspruch 1, dadurch gekennzeichnet, daß Y für Wasserstoff, Y' für Wasserstoff, Chlor oder Fluor stehen, n' 1 ist und X für $-C=O$ steht.

3. 2-Chlor-10,11-dihydro-1'-methyl-11-oxospiro[dibenz(b,f)oxepin-10,4'-piperidin] oder ein pharmazeutisch unbedenkliches Säureadditionssalz davon.

4. 2-Chlor-10,11-dihydro-11-oxospiro[dibenz(b,f)oxepin-10,4'-piperidin] oder ein pharmazeutisch unbedenkliches Säureadditionssalz davon.

5. 2-Chlor-10,11-dihydro-1'-äthyl-11-oxospiro[dibenz(b,f)oxepin-10,4'-piperidin] oder ein pharmazeutisch unbedenkliches Säureadditionssalz davon.

6. 1'-[3-(4-Fluorbenzoyl)-propyl]-10,11-dihydro-11-oxospiro[dibenz(b,f)oxepin-10,4'-piperidin] oder ein pharmazeutisch unbedenkliches Säureadditionssalz davon.

7. 10,11-Dihydro-11-oxo-1'-(2-propinyl)-spiro[dibenz(b,f)oxepin-10,4'-piperidin] oder ein pharmazeutisch unbedenkliches Säureadditionssalz davon.

8. 2-Chlor-1'-cyclopropylmethyl-10,11-dihydro-11-oxospiro[dibenz(b,f)oxepin-10,4'-piperidin] oder ein pharmazeutisch unbedenkliches Säureadditionssalz davon.

9. 2-Fluor-10,11-dihydro-11-oxospiro[dibenz(b,f)oxepin-10,4'-piperidin] oder ein pharmazeutisch unbedenkliches Säureadditionssalz davon.

10. 2-Fluor-10,11-dihydro-1'-methyl-11-oxospiro[dibenz(b,f)oxepin-10,4'-piperidin] oder ein pharmazeutisch unbedenkliches Säureadditionssalz davon.

11. Verbindung der Formel I zur Verwendung als Analgetikum, Tranquilizer oder Antikonvulsivum.

12. Verfahren zur Herstellung von Verbindungen der Formel I

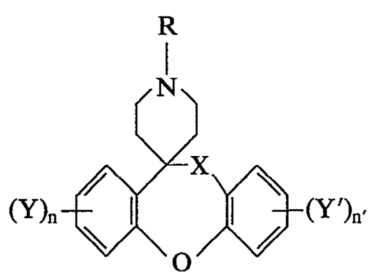

oder deren pharmazeutisch unbedenkliche Säureadditionssalze, worin R für Wasserstoff, $C_1-C_6$-Alkyl, $C_2-C_6$-Alkenyl, $C_2-C_6$-Alkinyl, $C_1-C_6$-Hydroxyalkyl, $C_3-C_7$-Cycloalkyl-$C_1-C_6$-alkyl, gegebenenfalls am Ring substituiertes Phenyl-$C_1-C_6$-alkyl, Phenoxy-$C_1-C_6$-alkyl oder Benzoyl-$C_1-C_6$-alkyl, wobei die Substituenten am Ring Nitro, Amino, Chlor, Fluor, Brom, Methoxy, $C_1-C_6$-Alkyl oder Trifluormethyl sein können, $C_2-C_6$-Alkanoyl, Cyan oder ein Äthylen-glykolketal der Formel

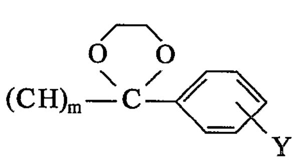

steht, X

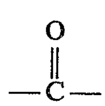 oder 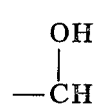

darstellt, wenn R die oben angegebene Bedeutung hat, oder

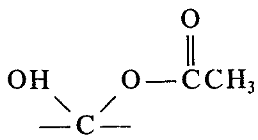

darstellt, wenn R für $C_2-C_6$-Alkanoyl, Methyl oder Cyan steht, Y und Y′ gleich oder verschieden sind und je Wasserstoff, Chlor, Fluor, Brom, Methoxy, Methylthio oder Trifluormethyl bedeuten können, n und n′ gleich oder verschieden sind und je eine ganze Zahl von 1 bis einschließlich 2 darstellen können und m eine ganze Zahl von 1 bis einschließlich 4 ist, dadurch gekennzeichnet, daß man

A) bzw. B) eine Verbindung der Formel

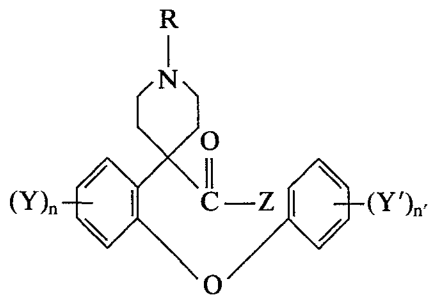

worin Y, Y′, n und n′ die zur Formel I genannten Bedeutungen haben, R für $C_1-C_6$-Alkyl oder $C_2-C_6$-Alkanoyl und Z für ein Halogenatom oder eine Hydroxygruppe steht, cyclisiert,

C) gegebenenfalls die erhaltene Verbindung der Formel I, worin R, Y, Y′, n und n′ die obengenannten Bedeutungen haben und X die Gruppe $-C=O$ bedeutet, sauer hydrolysiert zu einer Verbindung der Formel I, worin R Wasserstoff bedeutet,

D) gegebenenfalls eine Verbindung der Formel I, worin R Wasserstoff bedeutet, gemäß der ersten Stufe der Von-Braun-Reaktion mit Bromcyan umsetzt zu einer Verbindung der Formel I, worin R $-CN$ bedeutet, und gegebenenfalls die so erhaltene Verbindung gemäß der 2. Stufe der Von-Braun-Reaktion umsetzt zu einer Verbindung der Formel I, worin R Wasserstoff bedeutet,

E) gegebenenfalls eine Verbindung der Formel I, worin X die Gruppe $-C=O$ und R $-CN$, $C_1-C_6$-Alkyl oder $C_2-C_6$-Alkanoyl bedeutet, unter Friedel-Crafts-Bedingungen acyliert zu einer Verbindung der Formel I, worin X die Gruppe

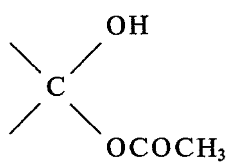

bedeutet,

F) gegebenenfalls eine Verbindung der Formel I, worin X die Gruppe

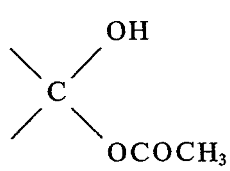

bedeutet, reduziert zu einer Verbindung der Formel I, worin X die Gruppe

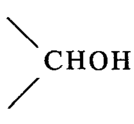

darstellt,

G) gegebenenfalls eine Verbindung der Formel I, worin R Wasserstoff bedeutet, alkyliert oder acyliert zu einer Verbindung der Formel I, worin R $C_1-C_6$-Alkyl, $C_1-C_6$-Hydroxyalkyl, $C_2-C_6$-Alkinyl, $C_2-C_6$-Alkenyl, Phenyl-$C_1-C_6$-alkyl, $C_3-C_7$-Cycloalkyl-$C_1-C_6$-alkyl, $C_2-C_6$-Alkanoyl oder Benzoyl-$C_1-C_6$-alkyl bedeutet,

H) gegebenenfalls eine Verbindung der Formel I, worin R Wasserstoff bedeutet, mit einem Chloräthylenglykolketal der Formel

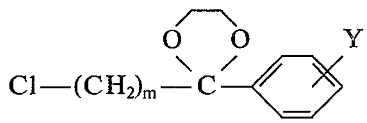

umsetzt zu einer Verbindung der Formel I, worin R die Benzoylalkyläthylenketalgruppe darstellt, und gegebenenfalls die so erhaltene Verbindung sauer hydrolysiert zu einer Verbindung der Formel I, worin R eine Benzoylniederalkylengruppe der Formel

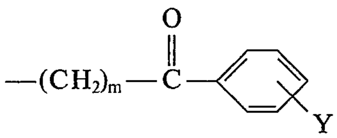

darstellt,

I) gegebenenfalls eine Verbindung der Formel I, worin R Wasserstoff bedeutet, gemäß den Bedingungen der Eschweiler-Clark-Reaktion umsetzt zu einer Verbindung der Formel I, worin R Methyl bedeutet, und

J) gegebenenfalls die erhaltenen Verbindungen der Formel I auf übliche Weise in ihre Säureadditionssalze überführt.

**Patentansprüche für den Vertragsstaat: AT**

1. Verfahren zur Herstellung von Verbindungen der Formel I

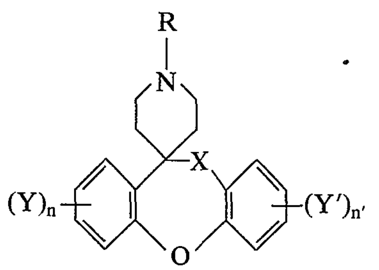

oder deren pharmazeutisch unbedenkliche Säureadditionssalze, worin R für Wasserstoff, $C_1-C_6$-Alkyl, $C_2-C_6$-Alkenyl, $C_2-C_6$-Alkinyl, $C_1-C_6$-Hydroxyalkyl, $C_3-C_7$-Cycloalkyl-$C_1-C_6$-alkyl, gegebenenfalls am Ring substituiertes Phenyl-$C_1-C_6$-alkyl, Phenoxy-$C_1-C_6$-alkyl oder Benzoyl-$C_1-C_6$-alkyl, wobei die Substituenten am Ring Nitro, Amino, Chlor, Fluor, Brom, Methoxy, $C_1-C_6$-Alkyl oder Trifluormethyl sein können, $C_2-C_6$-Alkanoyl, Cyan oder ein Äthylen-glykolketal der Formel

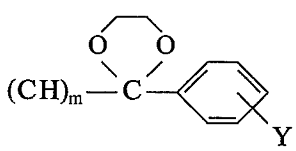

steht, X

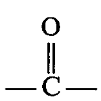

oder

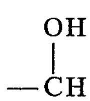

darstellt, wenn R die oben angegebene Bedeutung hat, oder

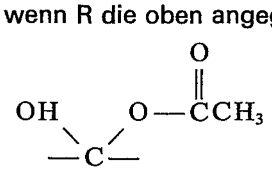

darstellt, wenn R für $C_2-C_6$-Alkanoyl, Methyl oder Cyan steht, Y und Y' gleich oder verschieden sind und je Wasserstoff, Chlor, Fluor, Brom, Methoxy, Methylthio oder Trifluormethyl bedeuten können, n und n' gleich oder verschieden sind und je eine ganze Zahl von 1 bis einschließlich 2 darstellen können und m eine ganze Zahl von 1 bis einschließlich 4 ist, dadurch gekennzeichnet, daß man

A) bzw. B) eine Verbindung der Formel

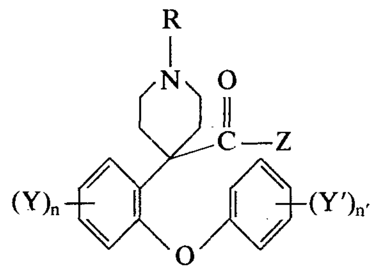

worin Y, Y', n und n' die zur Formel I genannten Bedeutungen haben, R für $C_1-C_6$-Alkyl oder $C_2-C_6$-Alkanoyl und Z für ein Halogenatom oder eine Hydroxygruppe steht, cyclisiert,

C) gegebenenfalls die erhaltene Verbindung der Formel I, worin R, Y, Y', n und n' die obengenannten Bedeutungen haben und X die Gruppe $-C=O$ bedeutet, sauer hydrolysiert zu einer Verbindung der Formel I, worin R Wasserstoff bedeutet,

D) gegebenenfalls eine Verbindung der Formel I, worin R Wasserstoff bedeutet, gemäß der ersten Stufe der Von-Braun-Reaktion mit Bromcyan umsetzt zu einer Verbindung der Formel I, worin R $-CN$ bedeutet, und gegebenenfalls die so erhaltene Verbindung gemäß der 2. Stufe der Von-Braun-Reaktion umsetzt zu einer Verbindung der Formel I, worin R Wasserstoff bedeutet,

E) gegebenenfalls eine Verbindung der Formel I, worin X die Gruppe $-C=O$ und R $-CN$, $C_1-C_6$-Alkyl oder $C_2-C_6$-Alkanoyl bedeutet, unter Friedel-Crafts-Bedingungen acyliert zu einer Verbindung der Formel I, worin X die Gruppe

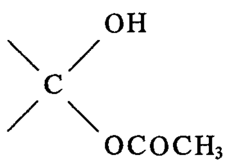

bedeutet,

F) gegebenenfalls eine Verbindung der Formel I, worin X die Gruppe

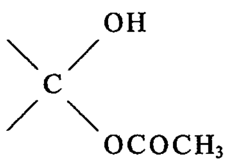

bedeutet, reduziert zu einer Verbindung der Formel I, worin X die Gruppe

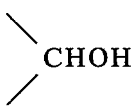

darstellt,

G) gegebenenfalls eine Verbindung der Formel I, worin R Wasserstoff bedeutet, alkyliert oder acyliert zu einer Verbindung der Formel I, worin R $C_1-C_6$-Alkyl, $C_1-C_6$-Hydroxyalkyl, $C_2-C_6$-Alkinyl, $C_2-C_6$-Alkenyl, Phenyl-$C_1-C_6$-alkyl, $C_3-C_7$-Cycloalkyl-$C_1-C_6$-alkyl, $C_2-C_6$-Alkanoyl oder Benzoyl-$C_1-C_6$-alkyl bedeutet,

H) gegebenenfalls eine Verbindung der Formel I, worin R Wasserstoff bedeutet, mit einem Chloräthylenglykolketal der Formel

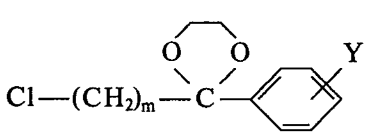

umsetzt zu einer Verbindung der Formel I, worin R die Benzoylalkyläthylenketalgruppe darstellt, und gegebenenfalls die so erhaltene Verbindung sauer hydrolysiert zu einer Verbindung der Formel I, worin R eine Benzoylniederalkylengruppe der Formel

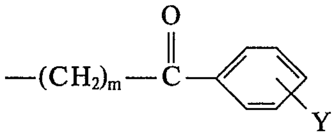

darstellt,

I) gegebenenfalls eine Verbindung der Formel I, worin R Wasserstoff bedeutet, gemäß den Bedingungen der Eschweiler-Clark-Reaktion umsetzt zu einer Verbindung der Formel I, worin R Methyl bedeutet, und

J) gegebenenfalls die erhaltenen Verbindungen der Formel I auf übliche Weise in ihre Säureadditionssalze überführt.

2. Verfahren zur Herstellung von Verbindungen der Formel I gemäß Anspruch 1, dadurch gekennzeichnet, daß Y für Wasserstoff, Y' für Wasserstoff, Chlor oder Fluor, X für $-C=O$ stehen und $n'=1$ ist.

**Claims for the contracting states: BE, CH, DE, FR, GB, IT, NL, SE**

1. A compound of the formula

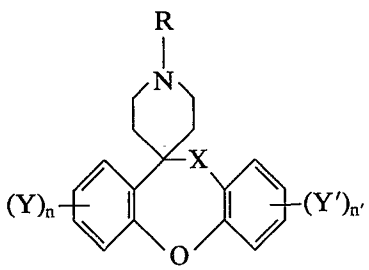

or a pharmaceutically acceptable acid addition salt thereof, in which R is hydrogen, $C_1-C_6$-alkyl, $C_2-C_6$-alkenyl, $C_2-C_6$-alkynyl, $C_1-C_6$-hydroxyalkyl, $C_3-C_7$-cycloalkyl-$C_1-C_6$-alkyl, phenyl-$C_1-C_6$-alkyl, phenoxy-$C_1-C_6$-alkyl, or benzoyl-$C_1-C_6$-alkyl, in each of which the phenyl ring may be substituted with nitro, amino, chlorine, fluorine, bromine, methoxy, $C_1-C_6$-alkyl or trifluormethyl, $C_2-C_6$-alkanoyl, cyano or ethylene glycol ketal of the formula

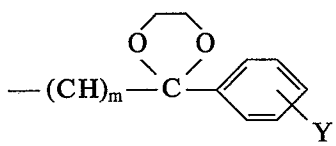

X is

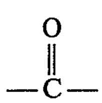 or 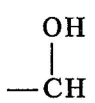

when R is as defined previously, or

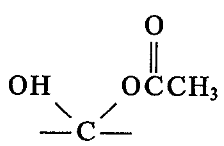

when R is $C_2-C_6$-alkanoyl, methyl or cyano; Y and Y' are the same or different and each can be hydrogen, fluorine, chlorine, bromine, methoxy, methylthio or trifluoromethyl; n and n' are the same or different and each can be an integer from 1 to 2, inclusive; and m is an integer from 1 to 4, inclusive.

2. A compound as defined in claim 1 in which Y is hydrogen, Y' is hydrogen, chlorine or fluorine and n' is 1 and X is $-C=O$.

3. The compound of claim 1 which is 2-chloro-10,11-dihydro-1'-methyl-11-oxospiro[dibenz(b,f)oxepin-10,4'-piperidine] or a pharmaceutically acceptable acid addition salt thereof.

4. The compound of claim 1 which is 2-chloro-10,11-dihydro-11-oxospiro[dibenz(b,f)oxepin-10,4'-piperidine] or a pharmaceutically acceptable acid addition salt thereof.

5. The compound of claim 1 which is 2-chloro-10,11-1'-ethyl-dihydro-11-oxospiro[dibenz(b,f)oxepin-10,4'-piperidine] or a pharmaceutically acceptable acid addition salt thereof.

6. The compound of claim 1 which is 1'-[3-(4-fluorobenzoyl)-propyl]-10,11-dihydro-11-oxopiro[dibenz(b,f)oxepin-10,4'-piperidine] or a pharmaceutically acceptable acid addition salt thereof.

7. The compound of claim 1 which is 10,11-dihydro-11'-oxo-1'-(2-propynyl)spiro[dibenz(b,f)oxepin-10,4'-piperidine] or a pharmaceutically acceptable acid addition salt thereof.

8. The compound of claim 1 which is 2-chloro-1'-cyclo-propylmethyl-10,11-dihydro-11-oxospiro[dibenz(b,f)oxepin-10,4'-piperidine] or a pharmaceutically acceptable acid addition salt thereof.

9. The compound of claim 1 which is 2-fluoro-10,11-dihydro-11-oxospiro[dibenz(b,f)oxepin-10,4'-piperidine] or a pharmaceutically acceptable acid addition salt thereof.

10. The compound of claim 1 which is 2-fluoro-10,11-dihydro-1'-methyl-11-oxospiro[dibenz(b,f)oxepin-10,4'-piperidine] or a pharmaceutically acceptable acid addition salt thereof.

11. A compound of the formula I as claimed in claim 1 for use as analgetic, tranquilizer or anticonvulsant agent.

12. A process for preparing a compound of the formula I

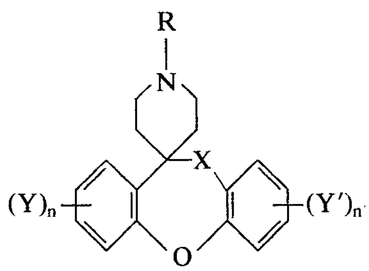

or a pharmaceutically acceptable acid addition salt thereof, in which R is hydrogen, $C_1-C_6$-alkyl, $C_2-C_6$-alkenyl, $C_2-C_6$-alkynyl, $C_1-C_6$-hydroxyalkyl, $C_3-C_7$-cycloalkyl-$C_1-C_6$-alkyl, phenyl-$C_1-C_6$-alkyl, phenoxy-$C_1-C_6$-alkyl, alkanoyl, or benzoyl-$C_1-C_6$-alkyl, in each of which the phenyl ring may be substituted with nitro, amino, chlorine, fluorine, bromine, methoxy, $C_1-C_6$-alkyl or trifluoromethyl, $C_2-C_6$-alkanoyl, cyano or ethylene glycol ketal of the formula

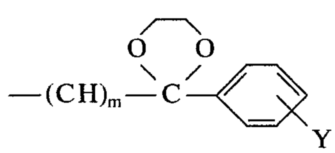

X is

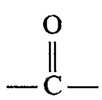 or 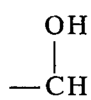

when R is as defined previously, or

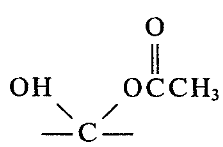

when R is $C_2-C_6$-alkanoyl, methyl or cyano; Y and Y' are the same or different and each can be hydrogen, chlorine, fluorine, bromine, methoxy, methylthio or trifluoromethyl; n and n' are the same or different and each can be an integer from 1 to 2, inclusive; and m is an integer from 1 to 4, inclusive, which comprises

A) or B), respectively, cyclizing a compound of the formula

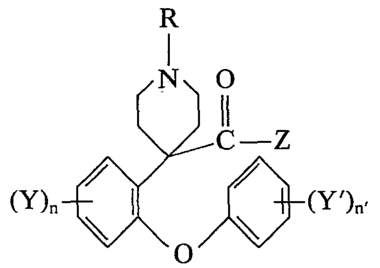

wherein Y, Y′, n and n′ are as defined in formula I, R is $C_1-C_6$-alkyl or $C_2-C_6$-alkanoyl and Z is halogen or hydroxy,

C) optionally subjecting the compound of the formula I wherein R, Y, Y′, n and n′ as as defined above and X is $-C=O$, to acid hydrolysis to yield a compound of the formula I wherein R is hydrogen,

D) optionally treating a compound of the formula I, wherein R is hydrogen, with cyanogen bromide in accordance with the first step of the von Braun reaction to form a compound of the formula I, wherein R is $-CN$, and optionally treating the compound so obtained in accordance with the second step of the von Braun reaction to form a compound of the formula I, wherein R is hydrogen,

E) optionally acylating a compound of the formula I, wherein X is $-C=O$ and R is $-CN$, $C_1-C_6$-alkyl or $C_2-C_6$-alkanoyl, under Friedel-Crafts-conditions to form a compound of the formula I, wherein X is the group

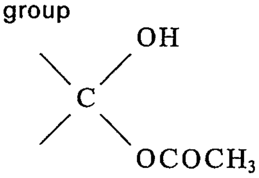

F) optionally reducing a compound of the formula I, wherein X is the group

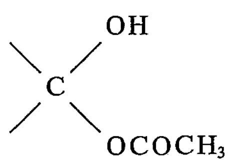

to form a compound of the formula I, wherein X is

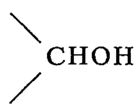

G) optionally alkylating or acylating a compound of the formula I, wherein R is hydrogen to form a compound of the formula I, wherein R is $C_1-C_6$-alkyl, $C_1-C_6$-hydroxyalkyl, $C_2-C_6$-alkynyl, $C_2-C_6$-alkenyl, phenyl-$C_1-C_6$-alkyl, $C_3-C_7$-cycloalkyl-$C_1-C_6$-alkyl, $C_2-C_6$-alkanoyl or benzoyl-$C_1-C_6$-alkyl,

H) optionally reacting a compound of the formula I, wherein R is hydrogen with a chloro ethylene glycol ketal of the formula

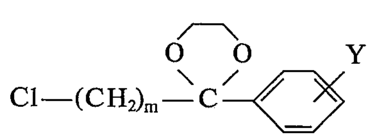

to form a compound of the formula I, wherein R is a benzoylalkylene ketal group and optionally subjecting the compound obtained to acid hydrolysis to provide the corresponding compound of the formula I, wherein R is a benzoylalkylene group of the formula

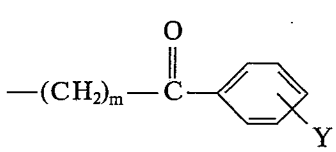

I) optionally reacting a compound of the formula I, wherein R is hydrogen, in accordance with the Eschwald-Clarke reaction to form a compound of the formula I wherein R is methyl, and

J) optionally preparing according to known methods the corresponding acid addition salts of the formula I.

**Claims for the contracting state: AT**

1. A process for preparing a compound of the formula I

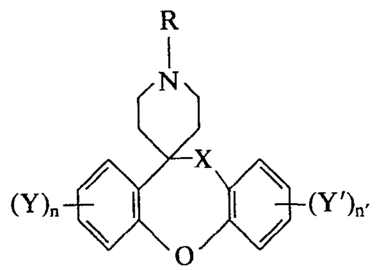

or a pharmaceutically acceptable acid addition salt thereof, in which R is hydrogen, $C_1-C_6$-alkyl, $C_2-C_6$-alkenyl, $C_2-C_6$-alkynyl, $C_1-C_6$-hydroxyalkyl, $C_3-C_7$-cycloalkyl-$C_1-C_6$-alkyl, phenyl-$C_1-C_6$-alkyl, phenoxy-$C_1-C_6$-alkyl, or benzoyl-$C_1-C_6$-alkyl, in each of which the phenyl ring may be substituted with nitro, amino, chlorine, fluorine, bromine, methoxy, $C_1-C_6$-alkyl or trifluoromethyl, $C_2-C_6$-alkanoyl, cyano or ethylene glycol ketal of the formula

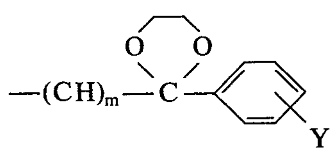

X is

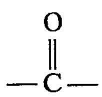 or 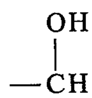

when R is as defined previously, or

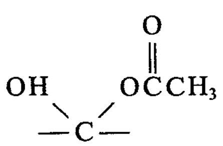

when R is $C_2-C_6$-alkanoyl, methyl or cyano; Y and Y′ are the same or different and each can be hydrogen, chlorine, fluorine, bromine, methoxy, methylthio or trifluoromethyl; n and n′ are the same or different and each can be an integer from 1 to 2, inclusive; and m is an integer from 1 to 4, inclusive, which comprises

A) or B), respectively, cyclizing a compound of the formula

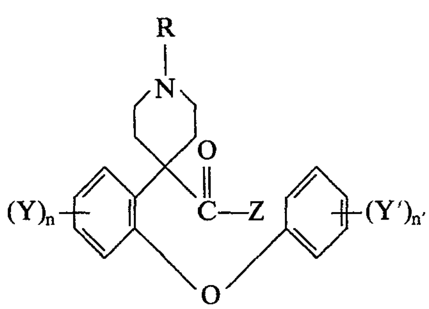

wherein Y, Y′, n and n′ are as defined in formula I, R is $C_1-C_6$-alkyl or $C_2-C_6$-alkanoyl and Z is halogen or hydroxy,

C) optionally subjecting the compound of the formula I wherein R, Y, Y', n and n' are as defined above and X is $-C=O$, to acid hydrolysis to yield a compound of the formula I wherein R is hydrogen,

D) optionally treating a compound of the formula I, wherein R is hydrogen, with cyanogen bromide in accordance with the first step of the von Braun reaction to form a compound of the formula I, wherein R is $-CN$, and optionally treating the compound so obtained in accordance with the second step of the von Braun reaction to form a compound of the formula I, wherein R is hydrogen,

E) optionally acylating a compound of the formula I, wherein X is $-C=O$ and R is $-CN$, $C_1-C_6$-alkyl or $C_2-C_6$-alkanoyl, under Friedel-Crafts-conditions to form a compound of the formula I, wherein X is the group

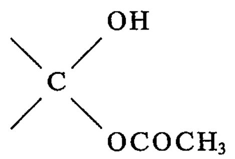

F) optionally reducing a compound of the formula I, wherein X is the group

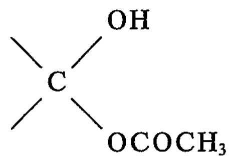

to form a compound of the formula I, wherein X is

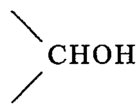

G) optionally alkylating or acylating a compound of the formula I, wherein R is hydrogen to form a compound of the formula I, wherein R is $C_1-C_6$-alkyl, $C_1-C_6$-hydroxyalkyl, $C_2-C_6$-alkynyl, $C_2-C_6$-alkenyl, phenyl-$C_1-C_6$-alkyl, $C_3-C_7$-cycloalkyl-$C_1-C_6$-alkyl, $C_2-C_6$-alkanoyl or benzoyl-$C_1-C_6$-alkyl,

H) optionally reacting a compound of the formula I, wherein R is hydrogen with a chloro ethylene glycol ketal of the formula

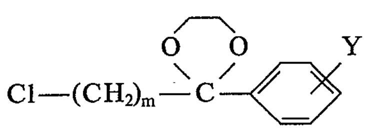

to form a compound of the formula I, wherein R is a benzoylalkylene ketal group and optionally subjecting the compound obtained to acid hydrolysis to provide the corresponding compound of the formula I, wherein R is a benzoylalkylene group of the formula

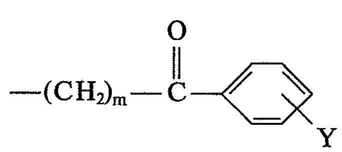

I) optionally reacting a compound of the formula I, wherein R is hydrogen, in accordance with the Eschwald-Clarke reaction to form a compound of the formula I wherein R is methyl, and

J) optionally preparing according to known methods the corresponding acid addition salts of the formula I.

2. A process as claimed in claim 1 which comprises preparing compounds of the formula I, wherein Y is hydrogen, Y' is hydrogen, chlorine or fluorine, X is $-C=O$ and n' is 1.

**Revendications pour les Etats contractants: BE, CH, DE, FR, GB, IT, NL, SE**

1. Composés répondant à la formule

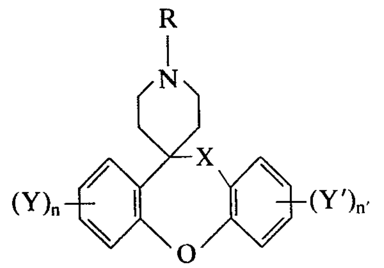

dans laquelle R représente l'hydrogène, un groupe alcoyle en $C_1-C_6$, alcényle en $C_2-C_6$, alcynyle en $C_2-C_6$, hydroxyàlcoyle en $C_1-C_6$, cycloalcoyl($C_3-C_7$)-alcoyle en $C_1-C_6$, phényl-alcoyle en $C_1-C_6$ éventuellement substitué sur le noyau, phénoxy-alcoyle en $C_1-C_6$ ou benzoyl-alcoyle en $C_1-C_6$, les substituants sur le noyau pouvant être des groupes nitro, amino, chloro, fluoro, bromo, méthoxy, alcoyle en $C_1-C_6$ ou trifluorométhyle, alcanoyle en $C_2-C_6$, cyano ou un éthylène-glycolcétal de formule

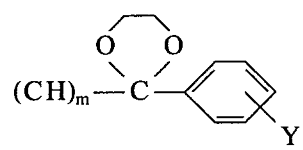

X représente

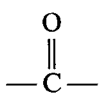 ou 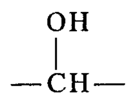

lorsque R a la signification indiquée ci-dessus, ou

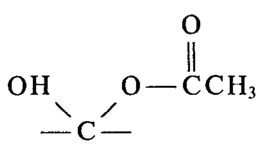

lorsque R représente un groupe alcanoyle en $C_2-C_6$, méthyle ou cyano, Y et Y' sont identiques ou différents et peuvent représenter chacun l'hydrogène, le chlore, le fluor, le brome, un groupe méthoxy, méthylthio ou trifluorométhyle, n et n' sont égaux ou différents et peuvent représenter chacun un nombre entier de 1 à 2 inclus et m est un nombre entrier de 1 à 4 inclus, ou leurs sels d'addition avec des acides pharmaceutiquement acceptables.

2. Composés selon la revendication 1, caractérisés en ce que Y représente l'hydrogène, Y' représente l'hydrogène, le chlore ou le fluor, n' est 1 et X représente le groupe

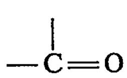

3. 2-Chloro-10,11-dihydro-1'-méthyl-11-oxospiro[dibenzo(b,f)oxépine-10,4'-pipéridine] ou un de ses sels d'addition avec un acide pharmaceutiquement acceptable.

4. 2-Chloro-10,11-dihydro-11-oxospiro[dibenzo(b,f)oxépine-10,4'-pipèridine] ou un de ses sels d'addition avec un acide pharmaceutiquement acceptable.

5. 2-Chloro-10,11-dihydro-1'-éthyl-11-oxospiro[dibenzo(b,f)oxépine-10,4'-pipéridine] ou un de ses sels d'addition avec un acide pharmaceutiquement acceptable.

6. 1'-[3-(4-Fluorobenzoyl)-propyl]-10,11-dihydro-11-oxospiro[dibenzo(b,f)oxépine-10,4'-pipéridine] ou un de ses sels d'addition avec un acide pharmaceutiquement acceptable.

7. 10,11-Dihydro-11-oxo-1'-(2-propinyl)-spiro[dibenzo(b,f)oxépine-10,4'-pipéridine] ou un de ses sels d'addition avec un acide pharmaceutiquement acceptable.

8. 2-Chloro-1'-cyclopropylméthyl-10,11-dihydro-11-oxospiro[dibenzo(b,f)oxépine-10,4'-pipéridine] ou un de ses sels d'addition avec un acide pharmaceutiquement acceptable.

9. 2-Fluoro-10,11-dihydro-11-oxospiro[dibenzo(b,f)oxépine-10,4'-pipéridine] ou un de ses sels d'addition avec un acide pharmaceutiquement acceptable.

10. 2-Fluoro-10,11-dihydro-1'-méthyl-11-oxospiro[dibenzo(b,f)oxépine-10,4'-pipéridine] ou un de ses sels d'addition avec un acide pharmaceutiquement acceptable.

11. Composé de formule I destiné à être utilisé comme analgésique, tranquillisant ou anticonvulsif.

12. Procédé de préparation de composés de formule I

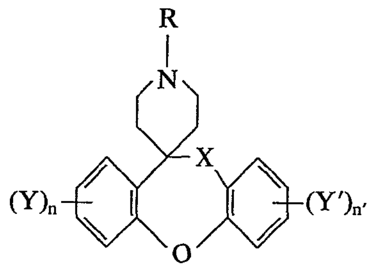

ou de leurs sels d'addition avec des acides pharmaceutiquement acceptables, dans laquelle formule R représente l'hydrogène, un groupe alcoyle en $C_1-C_6$, alcényle en $C_2-C_6$, alcynyle en $C_2-C_6$, hydroxyalcoyle en $C_1-C_6$, cycloalcoyl($C_3-C_7$)-alcoyle en $C_1-C_6$, phényl-alcoyle en $C_1-C_6$ éventuellement substitué sur le noyau phényle, phénoxy-alcoyle en $C_1-C_6$ ou benzoyl-alcoyle en $C_1-C_6$, les substituants sur le noyau pouvant être un groupe nitro, amino, chloro, fluoro, bromo, méthoxy, alcoyle en $C_1-C_6$ ou trifluorométhyle, alcanoyle en $C_2-C_6$, cyano ou un éthylène-glycolcétal de formule

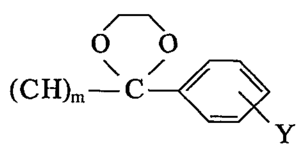

X représente un groupe

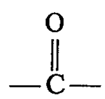

ou

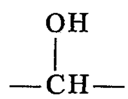

lorsque R a la signification indiquée ci-dessus ou

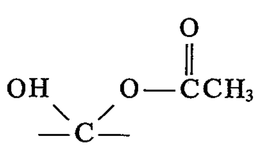

lorsque R représente un groupe alcanoyle en $C_2-C_6$, méthyle ou cyano, Y et Y' sont identiques ou différents et représentent chacun l'hydrogène, le chlore, le fluor, le brome, un groupe méthoxy, méthylthio ou trifluorométhyle, n et n' sont égaux ou différents et sont chacun un nombre entier de 1 à 2 inclus, et m est un nombre entier de 1 à 4 inclus, lequel procédé est caractérisé en ce qu'il consiste:

A) ou B) à cycliser un composé de formule

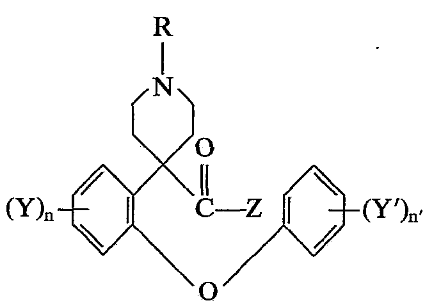

dans laquelle Y, Y', n et n' ont les significations indiquées dans la formule I, R représente un groupe alcoyle en $C_1-C_6$ ou alcanoyle en $C_2-C_6$ et Z est un atome d'halogène ou un groupe hydroxy,

C) éventuellement à hydrolyser en milieu acide le composé de formule I obtenu, où R, Y, Y', n et n' ont les significations indiquées ci-dessus et X représente le groupe

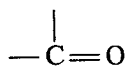

en un composé de formule I où R représente l'hydrogène,

D) éventuellement à faire réagir un composé de formule I où R représente l'hydrogène, avec du bromure de cyanogène selon le premier stade de la réaction de Von Braun pour obtenir un composé de formule I où R représente le groupe $-CN$, et éventuellement faire réagir le composé ainsi obtenu selon le second stade de la réaction de Von Braun pour obtenir un composé de formule I où R représente l'hydrogène,

E) éventuellement à acyler un composé de formule I, où X représente le groupe

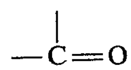

et R un groupe $-CN$, alcoyle en $C_1-C_6$ ou alcanoyle en $C_2-C_6$, dans des conditions de Friedel-Crafts, pour obtenir un composé de formule I où X représente le groupe

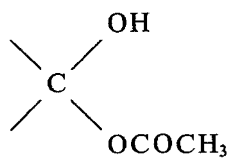

F) éventuellement à réduire un composé de formule I où X représente le groupe

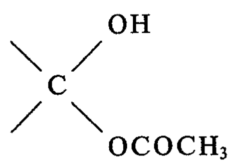

en un composé de formule I où X représente le groupe

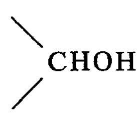

G) éventuellement à alcoyler ou acyler un composé de formule I où R représente l'hydrogène en un composé de formule I où R représente un groupe alcoyle en $C_1-C_6$, hydroxyalcoyle en $C_1-C_6$, alcynyle en $C_2-C_6$, alcényle en $C_2-C_6$, phényl-alcoyle en $C_1-C_6$, cycloalcoyl($C_3-C_7$)-alcoyle en $C_1-C_6$, alcanoyle en $C_2-C_6$ ou benzoyl-alcoyle en $C_1-C_6$,

H) éventuellement à faire réagir un composé de formule I où R représente l'hydrogène, avec un chloréthylène glycolacétal de formule

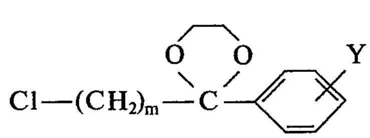

pour obtenir un composé de formule I où R représente le groupe benzoylalcoyléthylènecétal, et éventuellement hydrolyser en milieu acide le composé ainsi obtenu en un composé de formule I où R représente un groupe benzoylalkylène (inférieur) de formule

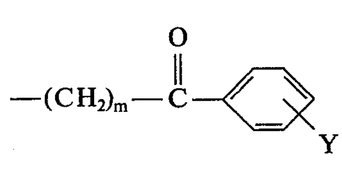

I) éventuellement à faire réagir un composé de formule I où R représente l'hydrogène selon les

conditions de la réaction d'Eschweiler-Clark pour obtenir un composé de formule I où R représente un groupe méthyle, et

J) éventuellement à convertir dùne façon usuelle les composés de formule I obtenus en leurs sels d'addition avec des acides.

**Revendications pour l'Etat contractant: AT**

1. Procédé de préparation de composés de formule I

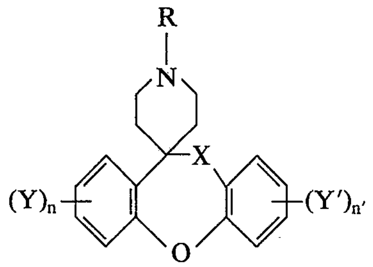

ou de leurs sels d'addition avec des acides pharmaceutiquement acceptables, dans laquelle formule: R représente l'hydrogène, un groupe alcoyle en $C_1-C_6$, alcényle en $C_2-C_6$, alcynyle en $C_2-C_6$, hydroxyalcoyle en $C_1-C_6$, cycloalcoyl($C_3-C_7$)-alcoyle en $C_1-C_6$, phénylalcoyle en $C_1-C_6$ éventuellement substitué sur le noyau phényle, phénoxy-alcoyle en $C_1-C_6$ ou benzoylalcoyle en $C_1-C_6$, les substituants sur le noyau pouvant être un groupe nitro, amino, chloro, fluoro, bromo, méthoxy, alcoyle en $C_1-C_6$ ou trifluorométhyle, alcanoyle en $C_2-C_6$, cyano ou éthylène-glycolcétal de formule

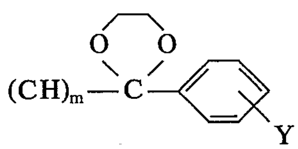

X représente un groupe

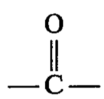

ou

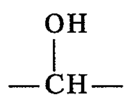

lorsque R a la signification indiquée ci-dessus ou

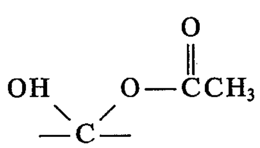

lorsque R représente un groupe alcanoyle en $C_2-C_6$, méthyle ou cyano, Y et Y' sont identiques ou différents et peuvent représenter chacun l'hydrogène, la chlore, le brome, le fluor, un groupe méthoxy, méthylthio ou trifluorométhyle, n et n' sont égaux ou différents et peuvent être chacun un nombre entier de 1 à 2 inclus, m est un nombre entier de 1 à 4 inclus, lequel procédé est caractérisé en ce qu'il consiste:

A) ou B) à cycliser un composé de formule

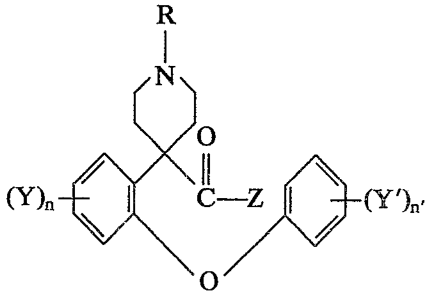

dans laquelle Y, Y', n et n' ont les significations indiquées pour la formule I, R représente un groupe alcoyle en $C_1-C_6$ ou alcanoyle en $C_2-C_6$ et Z est un atome d'halogène ou un groupe hydroxy,

C) éventuellement à hydrolyser en milieu acide la composé de formule I obtenu où R, Y, Y', n et n' ont les significations indiquées ci-dessus et X représente le groupe

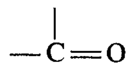

en un composé de formule I où R représente l'hydrogène,

D) éventuellement à faire réagir un composé de formule I où R représente l'hydrogène avec du bromure de cyanogène selon le premier stade de la réaction de Von Braun pour obtenir un composé de formule I où R représente le groupe $-CN$, et éventuellement faire réagir le composé ainsi obtenu selon le second stade de la réaction de Von Braun pour obtenir un composé de formule I où R représente l'hydrogène,

E) éventuellement à acyler un composé de formule I où X représente le groupe

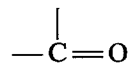

et R un groupe $-CN$, alcoyle en $C_1-C_6$ ou alcanoyle en $C_2-C_6$, dans des conditions de Friedel-Crafts pour obtenir un composé de formule I où X représente le groupe

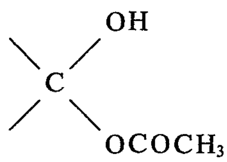

F) éventuellement à réduire un composé de formule I où X représente le groupe

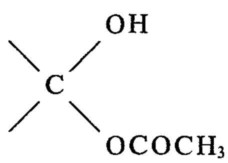

en un composé de formule I où X représente le groupe

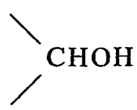

G) éventuellement à alcoyler ou acyler un composé de formule I où R représente l'hydrogène en un composé de formule I où R représente un groupe alcoyle en $C_1-C_6$, hydroxyalcoyle en $C_1-C_6$, alcynyle en $C_2-C_6$, alcényle en $C_2-C_6$, phénylalcoyle en $C_1-C_6$, cycloalcoyl($C_3-C_7$)-alcoyle en $C_1-C_6$, alcanoyle en $C_2-C_6$ ou benzoyl-alcoyle en $C_1-C_6$,

H) éventuellement à faire réagir un composé de formule I où R représente l'hydrogène avec un chloréthylène glycolcétal de formule

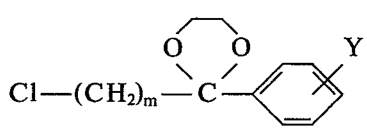

pour obtenir un composé de formule I où R représente le groupe benzoylalcoyléthylènecétal et éventuellement hydrolyser en milieu acide le composé ainsi obtenu en un composé de formule I où R représente un groupe benzoylalkylène (inférieur) de formule

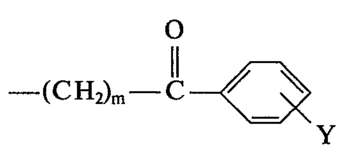

I) éventuellement à faire réagir un composé de formule I où R représente l'hydrogène, dans les conditions de la réaction d'Eschweiler-Clark, pour obtenir un composé de formule I où R représente un groupe méthyle, et

J) éventuellement à convertir d'une manière usuelle les composés de formule I obtenus en leurs sels d'addition avec des acides.

2. Procédé de préparation de composés de formule I selon la revendication 1, caractérisé en ce que Y représente l'hydrogène, Y' représente l'hydrogène, le chlore ou le fluor, X représente

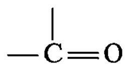

et n' est égal à 1.